(19) Europäisches Patentamt
European Patent Office
Office européen des brevets

(11) **EP 4 241 780 A1**

(12) **EUROPEAN PATENT APPLICATION**

(43) Date of publication:
**13.09.2023 Bulletin 2023/37**

(21) Application number: **22315053.3**

(22) Date of filing: **09.03.2022**

(51) International Patent Classification (IPC):
**A61K 36/73** (2006.01)   **A61P 39/06** (2006.01)

(52) Cooperative Patent Classification (CPC):
**A61K 36/73; A61P 39/06**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Diana**
**56250 Saint-Nolf (FR)**

(72) Inventor: **The designation of the inventor has not yet been filed**

(74) Representative: **De Crignis, Margot Gabrielle Diana**
**1, allée Ermengarde d'Anjou**
**35000 Rennes (FR)**

(54) **ENRICHED POLYPHENOLIC FRACTIONS OF ARONIA EXTRACTS AND USES THEREOF**

(57) The invention relates to specific enriched fractions of a polyphenolic extract of aronia. In particular, the invention related to proanthocyanidin-rich fractions (polymeric or oligomeric proanthocyanidins) and to anthocyanin/phenolic-rich fractions of an aronia extract. The invention also relates to compositions comprising such fractions and uses thereof, in particular in antioxidant or cytoprotective applications in nutraceutical, cosmetic or pharmaceutical compositions.

FIG. 4

Processed by Luminess, 75001 PARIS (FR)

**Description**

**Field of the invention**

The invention is in the field of natural extracts and their uses in cosmetic, nutraceutical, or pharmaceutic products. In particular, the invention relates to fractions of aronia extracts that are enriched in proanthocyanidins and in anthocyanins.

**Background**

In the past few decades, great attention has been focused to natural products sources of antioxidants as plants with bioactive compounds beneficial to human health. According to the "oxidative stress" theory, an imbalance between reactive oxygen species (ROS) and antioxidants can cause a loss of redox signaling and damage to DNA, proteins, and lipids, whose accumulation leads to aging and various degenerative and chronic diseases (Holmstrom and Finkel 2014, Rampon et al. 2018, Serino and Salazar 2018, Sies et al. 2017). Hence, an excess production of reactive species due to a stressor exposure (pollutants, exercise, injury, aging) is involved in the initiation and progression of several diseases. Under normal conditions, ROS are neutralized by antioxidant defense systems, some are synthetized in vivo (endogenous cellular antioxidant defenses including SOD, catalase, glutathione) and other come from our diet (exogenous phytochemicals or dietary antioxidants like vitamins C and E). Since higher fruit, vegetable and legume intake is associated with lower mortality in populations (Cassidy et al. 2013, Fraga et al. 2019, Miller et al. 2017), the importance of higher plant product consumption has received increased attention from the consumer. With more than 8000 known polyphenolic compounds being identified in the plant kingdom, these plant secondary metabolites are the most abundant dietary antioxidants (Manach et al. 2005, Pandey and Rizvi 2009, Tsao 2010).

Berry bushes such as black chokeberry (*Aronia melanocarpa*), are coming into the focus of researchers and the public, for health-promoting and chronic disease-preventing properties, particularly due to their great antioxidative activity (Borowska and Brzóska 2016, Jurikova et al. 2017, Sidor and Gramza-Michalowska 2019, Staszowska-Karkut and Materska 2020). Compared to other natural products, black chokeberry fruits are some of the richest sources of polyphenolic compounds, with a high content in phenolic acids (mostly chlorogenic and neochlorogenic acids) and flavonoids. Flavonoids present in aronia can be divided into four sub-groups: anthocyanins, flavonols, flavan-3-ols (or flavanols) and proanthocyanidins. Berries of aronia are known for their richness in anthocyanins (mostly cyanidin-3-galactoside and cyanidin-3-arabinoside) which give the dark color and can be used as food colorants. Aronia flavonols consist mainly in five quercetin derivatives (quercetin-3-O-galactoside, quercetin-3-O-glucoside, quercetin-3-O-rutinoside, quercetine-3-O-robinobioside and quercetin-3-O-vicianoside). Flavan-3-ols exist as monomeric forms (epicatechin), or oligomeric and polymeric forms (composed of epicatechin units) which are referred to as proanthocyanidins (PACs) (or called procyanidins when they consist exclusively of epicatechin units, like in aronia where they are type B procyanidins). One characteristic feature of aronia is the presence of high amount of polymeric forms of B-PACs with high degree of polymerization. Moreover, aronia contains active compounds in antioxidative function other than polyphenols, such as antioxidative vitamins (C and E), carotenoids, and minerals such as Zn, Cu, Mo and Se.

The "antioxidant properties" of bioactive compounds refer to several definitions or potential cellular mechanisms. Antioxidants, as agents that donate electrons or hydrogen atoms to oxidants, preventing them from oxidizing other molecules, exert direct effects including radical scavenging activity, suppression of the formation of reactive species, restoration of antioxidant enzyme and inhibition of prooxidant enzymes, or chelation of metal ions. Besides, biological effects elicited by indirect antioxidant properties of phytochemicals can be attributed to their ability to induce the redox sensitive transcription factor, Nuclear erythroid 2- related factor 2 (Nrf2) (Forman et al. 2014, Ma 2013). In response to homeostatic challenges, Nrf2 regulates the expression of about 250 cytoprotective genes that contain an enhancer sequence in their promoter region, the antioxidant response element (ARE). These genes encode for proteins involved in redox homeostasis, detoxification (xenobiotic biotransformation phase 2), heme metabolism, metabolism of carbohydrates and lipids, and regulators of inflammation (Ma 2013, Tebay et al. 2015). Nrf2 is regulated by Keap-1, an E3 ubiquitin ligase substrate adaptator which acts as a repressor by targeting Nrf2 for ubiquination and rapid proteasomal degradation. By reacting with highly reactive cysteine residues on Keap-1, electrophilic molecules prevent it from targeting Nrf2 for degradation and enable Nrf2 to accumulate in nucleus where it activates the ARE sequence. Many plant-derived compounds activate the Nrf2/Keap1/ARE1 pathway to elicit several cytoprotective responses, and discovery of new inducing agents are in the focus of biopharmaceutical companies for treating chronic diseases (Cuadrado et al. 2019).

Antioxidant properties of black chokeberries have been assessed both *in vitro* and *in vivo*. Aronia exerted a protective effect *in vivo* on animal models and *in vitro* on different cell lines modelling various diseases that have oxidative stress and inflammation as underlying feature, including antidiabetic, cardioprotective, hepatoprotective, antimutagenic, and anticarcinogenic effects (Denev et al. 2012). Anti-osteoclastogenic activity was reported, with an ethanolic extract (4% total polyphenols) or dry powder of aronia (2.6% polyphenols) inhibiting the ROS production in osteoclast cell models(Ghosh et al. 2018). An anthocyanin enriched extract from aronia attenuated the ROS production (LPS-induced) in human bronchial epithelial cells with reduction of the expression of inflammatory substrates and the production of inflammatory cytokines, suggesting anti-airway inflammation or respiratory disease protective effect (Jang et al. 2020). Also, a neuroprotective action was shown as anthocyanins from aronia inhibited ROS production and protected SH-SY5Y cells against Aβ1-42-induced apoptosis (Meng et al. 2018). *In vivo* animal studies have shown that an ethanolic extract of aronia exerted a gastroprotective activity on rat ulcer, which was mediated by an induction of different antioxidative enzymes (SOD, CAT, GPx) (Paulrayer et al. 2017). An aronia powder/extract has shown to be hepatoprotective in a mice model of alcohol-induced chronic liver injury with a reduction of lipid peroxidation and an increase in antioxidative enzyme levels, together with an activation of Nrf2 (Wang et al. 2020). Although it is more challenging to evaluate antioxidant activities occurring *in vivo* in humans, a reduction of DNA damage was reported in a pilot human study (n=10) on aronia juice consumption, but few changes of oxidative-related parameter could be measured (Nrf2, DOS, GPx, CAT, oxidized LDL) (Bakuradze et al. 2019). Xie et al. (2017) reported a reduction of LDL-Cholesterol (n=49) without changes in biomarkers of inflammation and oxidative stress after 12 weeks of a daily intake of 500 mg Aronia extract. Kardum et al. (2014) reported an increase in SOD and GPx activities after 3 months of consumption (n=25) of aronia juice (total polyphenols, 0.39%, 100mL/day for 3 months). Direct antioxidative activity of aronia was usually assessed using various chemical cell-free tests such as radical scavenging assays, effect of transition metals on changes in the state of oxidation, ability to inhibit lipid peroxidation. Intervention trials in humans with compositions comprising aronia extract have shown inconsistent results and generally failed to exert robust changes in global oxidative status (Kardum et al. 2014, Stankiewicz et al. 2021) and to positively modulate clinical biomarkers of conditions associated with oxidative stress such as cardiovascular disease (Ahles et al. 2020, Istas et al. 2019, Loo et al. 2016, Pokimica et al. 2019)or cognitive decline (Ahles et al. 2020).

An extensive review of the literature about the antioxidative properties of aronia assessed with all these different classical chemical test methods was published by Sidor and Gramza-Michalowska (2019): DPPH (22 references), ABTS (19 references), FRAP (14 references), ORAC (7 references), TRAP (3 references), lipid peroxidation (3 references), CUPRAC (3 references), including different measure units. The authors concluded that the antioxidative activity in black chokeberries was mainly attributed to polyphenols, with the main polyphenols contributing to the antioxidative effect of Aronia showing variation depending on the test method used. With DPPH, they were described as anthocyanins (66.7%), followed by proanthocyanidins (25%), flavonols and phenolic acids (8.2%), whereas with ORAC they were reported as anthocyanins (53%), phenolic acids (38.2%) and flavonols (8.7%) (proanthocyanidins not included in this study). All these studies were realized using aronia extracts, either crude or partially purified. Therefore, assessment of which particular polyphenol components were responsible for the observed biological effects could be difficult and often reported as a percentage of contribution relative to the whole content in the fruit.

It is now generally acknowledged that cell-free classical in vitro assessments are physiologically irrelevant, and the USDA removed its ORAC database for selected foods, citing "mounting evidence that the values indicating antioxidant capacity have no relevance to the effects of specific bioactive compounds, including polyphenols on human health" (Balentine et al. 2015). In European Union, the evaluation of health claim applications for botanical substances requires very stringent proof of efficacy. The EU commission however has authorized health allegations (listed in the Annex of the Commission Regulation (EU) N° 432/2012) for cocoa flavanols ("help maintain the elasticity of blood vessels, which contributes to normal blood flow") and olive oil polyphenols ("contribute to the protection of blood lipids from oxidative stress") (https://eur-lex.europa.eu/legal-content/EN/TXT/?uri=CELEX%3A02012R0432-20170822).

While the classical *in vitro* assessments are considered irrelevant, a need is growing toward plant-based solutions having a demonstrated antioxidant effect in reliable models. Innovative bioassays are now available to demonstrate the antioxidant effect of a compound or of a composition. Some are based on the LUCS technology (Derick et al. 2017) allowing for a quantitative measurement of ROS and/or free radical scavenging effects inside living cells as described by Gironde et al. (2020), and also by Vigliante et al. (2019) and Bianco et al. (2020).

However, these tools have never been used to measure the antioxidant effect of aronia, or of anthocyanin/phenolic-, oligomeric proanthocyanidins- or polymeric proanthocyanidins- rich fractions thereof. The inventors have now demonstrated that an aronia extract, and proanthocyanidins rich fractions thereof are particularly effective as antioxidant through a direct mechanism of action, whereas the anthocyanins rich fraction has a strong cytoprotective activity. These enriched fractions can be advantageously used in combination, in particular in reducing the oxidative stress and in preventing or treating diseases related to oxidative stress.

## Summary of the invention

An object of the invention relates to a proanthocyanidins (PACs) rich fraction of an aronia extract, said fraction comprising:

- an anthocyanin content of less than 5 dry wt.%, preferably less than 1 dry wt.%;

- a flavonol content of less than 1 dry wt.%, preferably less than 0.1 dry wt.%;
- a phenolic acid content of less than 0.1 dry wt.%;
- a PACs total content of at least 5 dry wt.%, preferably at least 10 dry wt.%.

The PACs total content, the anthocyanin content, the flavonol content and the phenolic acid are measured by liquid chromatographic techniques.

In an embodiment, the mean degree of polymerization of PACs is comprised between 5 and 15, and the fraction further comprises a flavan-3-ols total content of about 0.50 dry wt.%, as measured by liquid chromatographic techniques.

In another embodiment, the mean degree of polymerization of PACs is of at least 40, preferably at least 50, and the fraction further comprises a flavan-3-ols total content of less than 0.1 dry wt.% as measured by liquid chromatographic techniques. Moreover, the PACs total content is at least 30 dry wt.%, preferably at least 40 dry wt.%.

Another object according to the invention relates to an anthocyanin/phenolic-rich fraction of an aronia extract, said fraction comprising:

- an anthocyanin content of at least 5 dry wt.%, preferably of at least 8 dry wt.%;
- a flavonol content of at least 2.5 dry wt.%, preferably of at least 2.5 dry wt.%;
- a phenolic acid content of at least 15 dry wt.%, preferably of at least 20 dry wt.%;
- a flavan-3-ols total content of about 0.24 dry wt.%;
- a PACs total content of less than 5 dry wt.%, preferably of less than 2.5 dry wt.%;

wherein the anthocyanin content, the flavonol content, the phenolic acid, the flavan-3-ols total content and the PACs total content are measured by liquid chromatographic techniques.

According to a particular embodiment of these objects, the invention relates to the PACs- or anthocyanin/phenolic-rich fraction obtained from an aronia extract which is a polyphenolic aronia extract comprising PACs and anthocyanins, wherein:

- the polyphenol content is at least 55 dry wt.%, preferably at least 60 dry wt.%,
- the PACs content is at least 19 dry wt.%, preferably at least 20 dry wt.%,
- the PACs:anthocyanins ratio is at least 1.5, and

wherein the polyphenol content can be measured by the Folin-Ciocalteu (epicatechin equivalent), the PACs content can be measured by the BL-DMAC method (procyanidin A2 equivalent), and the anthocyanin content can be measured by spectrophotometry (cyanidin-3-O-glucoside chloride equivalent).

According to a preferred embodiment, said aronia extract is selected from the group consisting of an *Aronia arbutifolia* extract, an *Aronia melanocarpa* extract and an *Aronia prunifolia* extract, more preferably the aronia extract is an extract obtained from the fruits of *Aronia melanocarpa.* In a further embodiment, the aronia extract is in a dry powder form.

Another object according to the present invention related to a nutraceutical or cosmetic composition comprising, or consisting of, the PACs or anthocyanin rich fraction as described above.

The invention also relates to the use of such a composition, wherein the enriched fraction is a PACs enriched fraction, for preventing, limiting, or reducing the oxidative stress due to the exposure to pollutants, aging and/or exercise.

Another object according to the invention related to a pharmaceutical composition comprising the PACs- or anthocyanin/phenolic-rich fraction as described above. It is also recited such a pharmaceutical composition, wherein the enriched fraction is a PACs enriched fraction, for use in the treatment of a disorder related to cellular oxidative stress in a subject in need thereof. In an embodiment, the disorder related to cellular oxidative stress is selected from the group consisting of gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders.

The invention also relates to a pharmaceutical composition, wherein the enriched fraction is an anthocyanin/phenolic-rich fraction, for use as cytoprotective agent. Cytoprotective effects may include protection against oxidative damage to DNA, proteins and lipids, inflammation, mitochondrial dysfunction, and induction of antioxidant enzymes. These effects can occur at gastric, hepatic or cardiac level.

**Brief description of the figures**

Figure 1: Dose-response curves of an aronia extract and of three enriched fractions thereof according to the invention, as measured in AOP1 and AOPCAT assays in HepG2 cells. ACN: anthocyanin/phenolic-rich fraction; OLI: oligomeric PACs-rich fraction; POL: polymeric PACs-rich fraction.

Figure 2: Induction of the Nrf2/Keap1-mediated ARE pathway by an anthocyanin/phenolic-rich fraction (ACN) according to the invention. CTRL: control; Bars represent Standard Deviation.

Figure 3: Dose-response curve of an anthocyanin/phenolic-rich fraction (ACN) according to the invention, as measured in AOP2 assay in HepG2 cells.

Figure 4: $EC_{50}$ values for intracellular antioxidant activity of aronia crude extract, enriched fractions thereof according to the invention, other fruit powders and pure compounds.

## Detailed description

Definitions

In the context of the invention, "polyphenols" relates to molecules formed by more than one phenol unit. Flavonoids are the most commonly occurring polyphenols, and are, e.g., flavonols, flavanols, catechins, flavanones, anthocyanidins, isoflavonoids.

In the context of the invention, "proanthocyanidin" (PAC) or "proanthocyanidins" (PACs) or "condensed tannins" corresponds to oligomers or polymers of flavanols.

In the context of the invention, "anthocyanins" or "anthocyans" or "anthocyanosides" means glycosides of anthocyanidins, a subclass of flavonoids. The saccharide moiety of anthocyanins can be a monosaccharide (glucose, galactose, rhamnose), a disaccharide (rutinose, composed of a glucose linked to a rhamnose, xyloglucose) or at times a trisaccharide. Anthocyanins are water soluble plant pigments responsible for red, blue and purple colors found in many plants, including flowers and fruits.

In the context of the invention, "dry wt.%" refers to the percentage by weight of a compound relative to the total dry weight of an extract according to the invention unless specifically stated otherwise. For instance, an extract with a polyphenols content of 55 dry wt.% means that there is 55 g of polyphenols in 100 g of dry total extract. In another example, a fraction with an anthocyanin content of 10 dry wt.% means that there is 10 g of anthocyanins in 100 g of dry total fraction.

As used herein, "treatment" of a disorder includes the therapy or the prophylaxis of the disorder, or the prevention or delay in the onset of the disorder, or reduction of symptoms provoked by the disorder. The term treatment includes in particular the control of disease progression and associated symptoms.

As used herein, a "subject" refers to an individual receiving treatment. In one aspect, a subject can be a mammal. In another aspect, a subject can be a human. In another aspect, the subject can be a domesticated animal or livestock.

For the first time, the inventors have assessed the direct antioxidant effects of enriched fractions of an aronia extracts in innovative cell-based assays. Surprisingly, they have demonstrated that these direct effects are related to PACs (instead of a high anthocyanidin content), and that a cytoprotective effect is provided by the anthocyanin/phenolic-rich fraction.

Aronia extracts suitable in the context of the present invention

The aronia extracts suitable in the context of the present invention are obtained from plants of the genus *Aronia*. This genus encompasses shrubs known as chokeberries and belongs to the Rosaceae family. The main species of *Aronia* genus are *Aronia arbutifolia* (red aronia), *Aronia melanocarpa* (black chokeberry) or *Aronia prunifolia* (purple aronia). In a preferred embodiment, the aronia extract of the invention is an extract from *Aronia melanocarpa*.

The aronia extract suitable in the context of the present invention is rich in polyphenols. Methods for identifying and quantifying phenolics in extracts or compositions are known in the art and include, but are not limited to, for example, direct spectroscopy at 280 nm; indirect spectroscopy using, e.g., art recognized and commercially available reagents and assays, e.g., Vanillan assay, Folin-Denis assay, Folin-Ciocalteu assay, Prussian Blue assay, Bate-Smith assay, and Porter assay; and liquid chromatography, e.g., using ultraviolet, fluorescence, mass spectroscopy, and nuclear magnetic resonance (NMR). Phenolics detection techniques are also disclosed in the literature (see, e.g., Fereidoon and Naczk (1995)).

The polyphenol content of an aronia extract suitable in the context of the invention is of at least 55 dry wt.%, as it can be measured by the Folin Ciocalteu method and expressed as epicatechin equivalent. For example, extract according to the invention can contain at least 56 dry wt.%, at least 57 dry wt.%, at least 58 dry wt.%, at least 59 dry wt.%, at least 60 dry wt.%, at least 61 dry wt.% or at least 62 dry wt.% of total polyphenols, as it can be measured by the Folin Ciocalteu method and expressed as epicatechin equivalent. More preferably, the polyphenol content of the extract according to the invention is of at least 60 dry wt.%, as it can be measured by the Folin Ciocalteu method (epicatechin equivalent).

An aronia extract suitable in the context of the invention comprises about 5 dry wt.% of anthocyanins, e.g. about 4.5 dry wt.%, about 4.6 dry wt.%, about 4.7 dry wt.%, about 4.8 dry wt.%, about 4.9 dry wt.%, about 5.0 dry wt.%, about 5.1 dry wt.%, about 5.2 dry wt.%, about 5.3 dry wt.%, about 5.4 dry wt.%, or about 5.5 dry wt.% of anthocyanins, as measured by HPLC, in particular s measured by HPLC-Vis and expressed as Cyanidin 3-glucoside equivalent (Dudonné et al. 2014). Other methods that are well known in the art can be used to measure the anthocyanin content of the extract. For example, the anthocyanins content can be measured by spectrophotometry at 528 nm expressed as cyanidin-3-O-glucoside chloride equivalent and could therefore be at least 10 dry wt.%, preferably between 10 and 20 dry wt.%, more preferably between 10 and 15 dry wt.%.

An aronia extract suitable in the context of the invention comprises about 1.8 dry wt.% of flavonols, e.g. about 1.3 dry wt.%, about 1.4 dry wt.%, about 1.5 dry wt.%, about 1.6 dry wt.%, about 1.7 dry wt.%, about 1.8 dry wt.%, about 1.9 dry wt.%, about 2.0 dry wt.%, about 2.1 dry wt.%, about 2.2 dry wt.%, about 2.3 dry wt.% of flavonols, as measured by UPLC-UV-QToF and expressed as quercetin equivalent (Jakobek et al. 2013). Other well-known methods can be implemented to measure the flavonols content.

An aronia extract suitable in the context of the invention comprises about 11.50 dry wt.% of phenolic acids, e.g. about 11.25 dry wt.%, about 11.30 dry wt.%, about 11.35 dry wt.%, about 11.40 dry wt.%, about 11.45 dry wt.%, about 11.50 dry wt.%, about 11.55 dry wt.%, about 11.60 dry wt.%, about 11.65 dry wt.%, about 11.70 dry wt.%, about 11.75 dry wt.% of phenolic acids, as measured by UPLC-UV-QToF and expressed as chlorogenic equivalent (Jakobek et al. 2013). Other well-known methods can be implemented to measure the phenolic acids content.

An aronia extract suitable in the context of the invention comprises about 0.015 dry wt.% of catechin, e.g. about 0.010 dry wt.%, about 0.011 dry wt.%, about 0.012 dry wt.%, about 0.013 dry wt.%, about 0.014 dry wt.%, about 0.015 dry wt.%, about 0.016 dry wt.%, about 0.017 dry wt.%, about 0.018 dry wt.%, about 0.019 dry wt.%, about 0.020 dry wt.% of catechin, as measured by UPLC-UV-QToF (Jakobek et al. 2013). Other well-known methods can be implemented to measure the catechin content.

An aronia extract suitable in the context of the invention comprises about 0.125 dry wt.% of epicatechin, e.g. about 0.100 dry wt.%, about 0.105 dry wt.%, about 0.110 dry wt.%, about 0.115 dry wt.%, about 0.120 dry wt.%, about 0.125 dry wt.%, about 0.130 dry wt.%, about 0.135 dry wt.%, about 0.140 dry wt.%, about 0.145 dry wt.%, about 0.150 dry wt.% of epicatechin, as measured by UPLC-UV-QToF (Jakobek et al. 2013). Other well-known methods can be implemented to measure the epicatechin content.

An aronia extract suitable in the context of the invention comprises about 0.140 dry wt.% of total flavan-3-ols, e.g. about 0.115 dry wt.%, about 0.120 dry wt.%, about 0.125 dry wt.%, about 0.130 dry wt.%, about 0.135 dry wt.%, about 0.140 dry wt.%, about 0.145 dry wt.%, about 0.150 dry wt.%, about 0.155 dry wt.%, about 0.160 dry wt.%, about 0.165 dry wt.% of total flavan-3-ols, as measured by UPLC-UV-QToF and expressed as epicatechin equivalent (Jakobek et al. 2013). Other well-known methods can be implemented to measure the total flavan-3-ols content.

An aronia extract suitable in the context of the invention comprises at least 19 dry wt.% of total PACs, e.g. about 19.0 dry wt.%, about 19.5 dry wt.%, about 20.0 dry wt.%, about 20.5 dry wt.%, about 21.0 dry wt.%, about 21.5 dry wt.%, about 22.0 dry wt.%, about 22.5 dry wt.%, about 23.0 dry wt.%, about 23.5 dry wt.% or about 24.0 dry wt.% or more of total PACs, as measured by UPLC-UV-QToF (Jakobek et al. 2013). Other methods can be used to measure the PACs content, such as DMAC, BL-DMAC assay's (Prior et al. 2010) or adaptation thereof, such as BL-DMAC expressed as procyanidin A2 equivalent, phloroglucinolysis, the European Pharmacopeia or adaptation thereof.

A preferred aronia extract suitable in the context of the invention has a polyphenol content of at least 55 dry wt.%, preferably at least 60 dry wt.%, as measured by the Folin Ciocalteu (epicatechin equivalent), a PACs content of at least 19 dry wt.%, preferably at least 20 dry wt.%, as measured by the BL-DMAC method (procyanidin A2 equivalent), and a PACs:anthocyanins ratio of at least 1.5, with the anthocyanin content being measured by spectrophotometry (cyanidin 3-O-glu Cl equivalent).

The PACs of an aronia extract suitable in the context of the invention have a mean degree of polymerization of about 30, e.g. about 27, about 28, about 29, about 30, about 31, about 32, or about 33. The degree of polymerization can be measured by UPLC-UV-QToF (Jakobek et al. 2013).

Process for obtaining an aronia extract suitable in the context of the present invention

An aronia extract suitable in the context of the invention can be obtained by phenolic extraction from a plant or part of a plant of *Aronia* genus. A part of a plant means the leaf or flowers or fruits, in a solid or liquid form (solid or liquid substrate).

In a preferred embodiment, the aronia extract suitable in the context of the invention is obtained by phenolic extraction from a liquid substrate, such as a juice or a concentrated juice of plants or part of a plant of *Aronia* genus, preferably a juice of fruits (berries) from a plant of *Aronia* genus, more preferably a concentrated juice of fruits (berries) from a plant of *Aronia* genus.

In a very preferred embodiment, the aronia extract suitable in the context of the invention is obtained by phenolic extraction from a concentrated juice of fruits of *Aronia melanocarpa*, and more preferably from a concentrated juice of fruits of *Aronia melanocarpa* comprising at least 0.5% (w/w) of anthocyanins at 65 Brix (cyanidin-3-O-glucoside chloride equivalent).

The preferred method for extracting polyphenols is chromatography extraction. In this embodiment, a solid substrate is diluted to become liquid, and a liquid substrate can advantageously be diluted. In a preferred embodiment, the substrate is diluted until a Brix value from about 20 to about 30, such as about 20°Brix, about 21°Brix, about 22°Brix, about 23°Brix, about 24°Brix, about 25°Brix, about 26°Brix, about 27°Brix, about 28°Brix, about 29°Brix or about 30°Brix, is reached. More preferably, a concentrated juice of fruits of aronia comprising at least 0.5% (w/w) of anthocyanins at 65° Brix (cyanidin -3-O-glucoside chloride equivalent) is diluted until reaching a Brix of about 25. The dilution can be made with known solutions, such as purified water.

The diluted substrate is then loaded in the chromatography column. The preferred resin for the method for extracting polyphenols is Amberlite™ XAD-7HP resin (distributed by The Dow Chemical). XAD-7HP is a commercially available macroreticular aliphatic crosslinked polymer ester resin that consists of white translucent beads that have a high surface area of at least 300 $m^2$/g, i.e. of about 500 $m^2$/g, an average pore diameter of about 550 Angstroms, a total pore volume of about 0.5 mL/mL, a water retention capacity of 61 to 69%, a particle diameter of 430 to 690 $\mu$m, less than.7.0% of particles with a size under 300 $\mu$m, less than 8.0% of particles having a size above 1180 $\mu$m and a particle density from 1.06 to 1.08 g/mL.

More preferably, the resin is first conditioned with purified water. Purified water includes osmosed water, and preferably acidified osmosed water. Osmosed water can be acidified by adding an acid solution to osmosed water. In a preferred embodiment, acidified water is prepared by adding $H_2SO_4$ solution (about 96% v/v) to osmosed water to a final concentration of about 0.03% (v/v). In a preferred embodiment, the resin is conditioned at a pH equal to or of less than 3.5. In a more preferred embodiment, Amberlite™ XAD-7HP resin is conditioned at a pH equal to or of less than 3.5 with acidified water prepared by adding $H_2SO_4$ solution (96% v/v) to osmosed water to a final concentration of 0.03% (v/v).

The amount of resin used can be adapted and depend upon the scale of the process and/or the volume or capacity of the column.

The time and conditions sufficient for phenolic compounds present in the composition to bind the resin include those times and conditions under which the desirable quantity of phenolics present in the composition bind to the resin.

The substrate loaded in the chromatography resin can then be washed to eliminate sugars and some acids. Washing solutions that can be used in the process of the invention can include water-based solutions, such as pure water or solutions containing water and another compound, such as a solvent or an acid. In a preferred embodiment, purified water is used, and preferably acidified water such as described above. The washing step can be performed by the addition of 1 to 5 BV (Bed Volume) of acidified water to the column, such as 1 BV, 2 BV, 3 BV, 4 BV or 5 BV of acidified water. Preferably, the washing step is performed by the addition of 2 to 3 BV of acidified water.

The substrate loaded in the chromatography column can then be eluted to recover polyphenols that have bound to the resin. Elution solutions that can be used in the process of the invention can include water-based solutions containing one or more solvents at any concentration that will increase the release of polyphenols from the resin. In a preferred embodiment, the elution solution consists of an acidified hydro-ethanolic solution, such as a solution comprising from 1% to 99% v/v of ethanol and 99% to 1% of acidified water. A preferred acidified hydro-ethanolic solution comprises from about 30% to about 70% of ethanol and from about 70% to about 30% of acidified water, e.g. about 30% of ethanol and about 70% of acidified water, about 40% of ethanol and about 60% of acidified water, about 50% of ethanol and about 50% of acidified water, about 60% of ethanol and about 40% of acidified water, or about 70% of ethanol and about 30% of acidified water.

The elution flow is collected. The elution flow is is a polyphenolic aronia extract comprising PACs and anthocyanins, wherein:

- the polyphenol content is at least 55 dry wt.%, preferably at least 60 dry wt.%,
- the PACs content is at least 19 dry wt.%, preferably at least 20 dry wt.%,
- the PACs:anthocyanins ratio is at least 1.5, and

wherein the polyphenol content can be measured by the Folin-Ciocalteu (epicatechin equivalent), the PACs content can be measured by the BL-DMAC method (procyanidin A2 equivalent), and the anthocyanin content can be measured by spectrophotometry (cyanidin-3-O-glucoside chloride equivalent).

The elution flow can optionally be further processed.

For instance, solvent of the elution solution can be partially or totally evaporated. In a preferred embodiment, the elution flow is concentrated by evaporation, until a dry matter content from 35 to 55 % w/w is reached, preferably from 40 to 50 % w/w, such as about 35% w/w, about 36% w/w, about 37% w/w, about 38% w/w, about 39% w/w, about 40% w/w, about 41% w/w, about 42% w/w, about 43% w/w, about 44% w/w, about 45% w/w, about 46% w/w, about 47% w/w, about 48% w/w, about 49% w/w, about 50% w/w, about 51% w/w, about 52% w/w, about 53% w/w, about 54% w/w or about 55% w/w. Evaporation can be implemented at a temperature from 60 °C to 75°C, preferably at a temperature from 68°C to 72°C.

The concentrated elution flow can be dried, i.e., further concentrated until a higher dry matter content is reached, to obtain a dry extract. Suitable concentration methods include, but are not limited to, membrane concentration, heat concentration, vacuum (reduced pressure) concentration, and freeze concentration. In a preferred embodiment, the concentrated elution flow is dried by vacuum drying.

In a preferred embodiment, the dry extract is grinded to make a powder. The powder can advantageously be sieved. The mesh size for sieving can be from about 250 $\mu$m to about 1000 $\mu$m, preferably of about 500 $\mu$m.

According to a preferred embodiment, the dry powdered extract is packaged. This embodiment is advantageous for selling the extract as an ingredient, for instance as an ingredient for nutraceutical, cosmetic or pharmaceutic products.

Process for preparing an enriched phenolic fraction of an aronia extract

The invention related to polyphenol rich fractions of an aronia extract, namely an anthocyanin/phenolic-rich fraction (referred to as "ACN"), an oligomeric PACs rich fraction (referred to as "OLI") and a polymeric PACs rich fraction (referred to as "POL"). These fractions can be obtained from an aronia extract, preferably from an aronia polyphenolic extract as described above, by know methods such as chromatographic separation. In a preferred embodiment, the fractions rich in anthocyanin and phenolic acid, in oligomeric PACs and in polymeric PACs are separated on a gel-filtration chromatography, such as the method described by Rodríguez-Daza et al. (2020) or adaptations thereof.

ACN

An anthocyanin/phenolic-rich fraction of an aronia extract according to the invention comprises at least 5 dry wt.% of anthocyanins, preferably at least 8 dry wt.%, e.g. about 8.0 dry wt.%, about 8.5 dry wt.%, about 9.0 dry wt.%, about 9.5 dry wt.%, about 10.0 dry wt.%, about 10.5 dry wt.%, about 11.0 dry wt.%, about 11.5 dry wt.%, about 12.0 dry wt.%, about 12.5 dry wt.%, or about 13.0 dry wt.% or more of anthocyanins, as measured by liquid chromatography techniques, such as HPLC, in particular as measured by HPLC-Vis and expressed as Cyanidin 3-glucoside equivalent (Dudonné et al. 2014). Other methods that are well known in the art can be used to measure the anthocyanin content of the extract.

An anthocyanin/phenolic-rich fraction of an aronia extract according to the invention comprises at least 2 dry wt.%, preferably at least 2.5 dry wt.%, even more preferably of about 3.65 dry wt.% of flavonols, e.g. about 3.40 dry wt.%, about 3.45 dry wt.%, about 3.50 dry wt.%, about 3.55 dry wt.%, about 3.60 dry wt.%, about 3.65 dry wt.%, about 3.70 dry wt.%, about 3.75 dry wt.%, about 3.80 dry wt.%, about 3.85 dry wt.%, about 3.90 dry wt.% of flavonols, as measured by liquid chromatography techniques, such as UPLC, in particular as measured by UPLC-UV-QToF and expressed as quercetin equivalent (Jakobek et al. 2013). Other well-known methods can be implemented to measure the flavonols content.

An anthocyanin/phenolic-rich fraction of an aronia extract according to the invention comprises at least 15 dry wt.% of phenolic acids, preferably at least 20 dry wt.%, e.g. about 20.0 dry wt.%, about 20.5 dry wt.%, about 21.0 dry wt.%, about 21.5 dry wt.%, about 22.0 dry wt.%, about 22.5 dry wt.%, about 23.0 dry wt.%, about 23.5 dry wt.%, or more of phenolic acids, as measured by liquid chromatography techniques, such as UPLC, in particular as measured by UPLC-UV-QToF and expressed as chlorogenic equivalent (Jakobek et al. 2013). Other well-known methods can be implemented to measure the phenolic acids content.

An anthocyanin/phenolic-rich fraction of an aronia extract according to the invention comprises about 0.24 dry wt.% of total flavan-3-ols, e.g. about 0.20 dry wt.%, about 0.21 dry wt.%, about 0.22 dry wt.%, about 0.23 dry wt.%, about 0.24 dry wt.%, about 0.25 dry wt.%, about 0.26 dry wt.%, about 0.27 dry wt.%, or about 0.28 dry wt.% of total flavan-3-ols, as measured by liquid chromatography techniques, such as UPLC, in particular as measured by UPLC-UV-QToF and expressed as epicatechin equivalent (Jakobek et al. 2013). Other well-known methods can be implemented to measure the total flavan-3-ols content.

An anthocyanin/phenolic-rich fraction of an aronia extract according to the invention comprises less than 5 dry wt.% of total PACs, preferably of less than 2.5 dry wt.% of total PACs, as measured by liquid chromatography techniques, such as UPLC, in particular as measured by UPLC-UV-QToF (Jakobek et al. 2013). Other methods can be used to measure the total PACs content, such as DMAC, BL-DMAC assay's (Prior et al. 2010) or adaptation thereof, such as BL-DMAC expressed as procyanidin A2 equivalent, phloroglucinolysis, the European Pharmacopeia or adaptation thereof.

The PACs of an anthocyanin/phenolic-rich fraction of an aronia extract according to the invention have a mean degree of polymerization of about 3, e.g. 2, 3, or 4 as determined by liquid chromatography techniques, such as UPLC, in particular as measured by UPLC-UV-QToF (Jakobek et al. 2013).

OLI

An oligomeric PACs- rich fraction of an aronia extract according to the invention comprises less than 5 dry wt.% of anthocyanins, preferably less than 1 dry wt.%, more preferably less than 0.5 dry wt.% of anthocyanins, as measured by liquid chromatography techniques, such as HPLC, in particular as measured by HPLC-Vis and expressed as Cyanidin 3-glucoside equivalent (Dudonné et al. 2014). Other methods that are well known in the art can be used to measure the anthocyanin content of the extract.

An oligomeric PACs- rich fraction of an aronia extract according to the invention comprises less than 1 dry wt.% of flavonols, preferably less than 0.1 dry wt.%, more preferably less than 0.05 dry wt.% of flavonols as measured by liquid chromatography techniques, such as UPLC, in particular as measured by UPLC-UV-QToF and expressed as quercetin equivalent (Jakobek et al. 2013). Other well-known methods can be implemented to measure the flavonols content.

An oligomeric PACs- rich fraction of an aronia extract according to the invention comprises less than 0.1 dry wt.% of phenolic acids, as measured by liquid chromatography techniques, such as UPLC, in particular as measured by UPLC-UV-QToF and expressed as chlorogenic equivalent (Jakobek et al. 2013). Other well-known methods can be implemented to measure the phenolic acids content.

An oligomeric PACs- rich fraction of an aronia extract according to the invention comprises about 0.50 dry wt.% of total flavan-3-ols, e.g. about 0.45 dry wt.%, about 0.46 dry wt.%, about 0.47 dry wt.%, about 0.48 dry wt.%, about 0.49 dry wt.%, about 0.50 dry wt.%, about 0.51 dry wt.%, about 0.52 dry wt.%, about 0.53 dry wt.%, about 0.54 dry wt.%, or about 0.55 dry wt.% of total flavan-3-ols, as measured by liquid chromatography techniques, such as UPLC, in particular as measured by UPLC-UV-QToF and expressed as epicatechin equivalent (Jakobek et al. 2013). Other well-known methods can be implemented to measure the total flavan-3-ols content.

An oligomeric PACs- rich fraction of an aronia extract according to the invention comprises at least 5 dry wt.% of total PACs, preferably at least 6 dry wt.%, more preferably at least 10 dry wt.%, and even more preferably about 15 dry wt.% of total PACs as measured by liquid chromatography techniques, such as UPLC, in particular as measured by UPLC-UV-QToF (Jakobek et al. 2013). Other methods can be used to measure the total PACs content, such as DMAC, BL-DMAC assay's (Prior et al. 2010) or adaptation thereof, such as BL-DMAC expressed as procyanidin A2 equivalent, phloroglucinolysis, the European Pharmacopeia or adaptation thereof.

The PACs of an oligomeric PACs- rich fraction of an aronia extract according to the invention have a mean degree of polymerization comprised between 5 and 15, e.g. about 6, about 7, about 8, about 9, about 10, about 11, about 12, about 13, or about 14, as determined by liquid chromatography techniques, such as UPLC, in particular as measured by UPLC-UV-QToF (Jakobek et al. 2013).

POL

A polymeric PACs- rich fraction of an aronia extract according to the invention comprises less than 5 dry wt.% of anthocyanins, preferably less than 1 dry wt.%, more preferably less than 0.5 dry wt.% as measured by liquid chromatography techniques, such as HPLC, in particular as measured by HPLC-Vis and expressed as Cyanidin 3-glucoside equivalent (Dudonné et al. 2014). Other methods that are well known in the art can be used to measure the anthocyanin content of the extract.

A polymeric PACs- rich fraction of an aronia extract according to the invention comprises less than 1 dry wt.% of flavonols, preferably less than 0.1 dry wt.%, more preferably less than 0.05 dry wt.% of flavonols as measured by liquid chromatography techniques, such as UPLC, in particular as measured by UPLC-UV-QToF and expressed as quercetin equivalent (Jakobek et al. 2013). Other well-known methods can be implemented to measure the flavonols content.

A polymeric PACs- rich fraction of an aronia extract according to the invention comprises less than 0.1 dry wt.% of phenolic acids, preferably less than 0.5 dry wt.% of phenolic acids, as measured by liquid chromatography techniques, such as UPLC, in particular as measured by UPLC-UV-QToF and expressed as chlorogenic equivalent (Jakobek et al. 2013). Other well-known methods can be implemented to measure the phenolic acids content.

A polymeric PACs- rich fraction of an aronia extract according to the invention comprises less than 0.1 dry wt.% of total flavan-3-ols, preferably about 0.080 dry wt.% of total flavan-3-ols, as measured by liquid chromatography techniques, such as UPLC, in particular as measured by UPLC-UV-QToF and expressed as epicatechin equivalent (Jakobek et al. 2013). Other well-known methods can be implemented to measure the total flavan-3-ols content.

A polymeric PACs- rich fraction of an aronia extract according to the invention comprises at least 30 dry wt.% of total PACs, preferably at least 40 dry wt.% of total PACs, more preferably about 50 dry wt.% of total PACs, as measured by liquid chromatography techniques, such as UPLC, in particular as measured by UPLC-UV-QToF (Jakobek et al. 2013). Other methods can be used to measure the total PACs content, such as DMAC, BL-DMAC assay's (Prior et al. 2010) or adaptation thereof, such as BL-DMAC expressed as procyanidin A2 equivalent, phloroglucinolysis, the European Pharmacopeia or adaptation thereof.

The PACs of a polymeric PACs- rich fraction of an aronia extract according to the invention have a mean degree of polymerization of at least 40, preferably at least 50, more preferably of at least 60, and even more preferably of about 75, as determined by liquid chromatography techniques, such as UPLC, in particular as measured by UPLC-UV-QToF (Jakobek et al. 2013).

Composition and uses thereof

According to another object, the invention relates to compositions comprising at least one of the enriched fractions (ACN, OLI, POL) of the aronia extract as described above.

It includes compositions which do not comprise any other compound than those of the enriched fraction(s) according to the invention. Hence, in an embodiment, the invention related to compositions consisting of at least one of the enriched fractions according to the invention, such as a composition consisting of OLI, a composition consisting of POL, a composition consisting of OLI and POL, a composition consisting of ACN, a composition consisting of ACN and OLI, a composition consisting of ACN and POL, or a composition consisting of OLI, POL and ACN. Such "pure" compositions can be advantageous because the polyphenols of interest are not diluted with other compounds and could be more effective. It is also an interesting product for the manufacturers, who can use the enriched fractions of the invention according to their own recipes or composition.

In another embodiment, the composition comprises at least one of the enriched fractions according to the invention with at least one other compound. For example, a composition according to the invention can comprise at least one of the enriched fractions as described above and at least one additional compound, such as at least one additional compound selected from the group consisting of sweeteners, preservatives, flavoring agents, thickeners, coatings, isotonic agents, absorption delaying agents, binders, adhesives, lubricants, disintegrants, coloring agents, absorbents, detergents, emulsifying gents, antioxidants, vitamins, minerals, proteins, fats, carbohydrates, and also food matrix such as fruits, vegetables or meat preparations, or a combination thereof. Consequently, in a preferred embodiment, the composition comprising the aronia extract is a food, nutraceutical or cosmetic composition, depending on the additional compound(s) used in the composition.

POL and OLI have shown to be particularly effective for preventing, limiting, or reducing the intracellular oxidative stress in cells (see Example 3). Hence, a composition consisting of, or comprising, POL and/or OLI fractions according to the present invention can be useful for preventing, limiting, or reducing the oxidative stress due to the exposure to pollutants, aging and/or exercise.

Because of the unexpected direct antioxidant effect of POL and OLI fractions according to the present invention, other objects of the invention relate to a pharmaceutical composition comprising POL and/or OLI according to the invention and to such a pharmaceutical composition for use in the treatment of a disorder related to cellular oxidative stress in a subject in need thereof. Disorders related to cellular oxidative stress are well known in the art and there is a considerable literature that deals with this. In a preferred embodiment, the disorder related to cellular oxidative stress is selected from the group consisting of gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders.

On the other hand, ACN has demonstrated a cytoprotective effect through the activation of the Nrf2-Keap1 pathway (cf. Example 3). Hence, a composition consisting of, or comprising ACN according to the invention can be useful in protecting the cells from external stresses and harmful agents.

A pharmaceutical composition comprising at least one of the enriched fractions according to the invention typically comprises one or several pharmaceutically acceptable carriers or excipients. The dosage, frequency and mode of administration of the pharmaceutical composition of the invention and the duration of the therapy depends on the stage of the disease being treated, the age and condition of the patient, and how the patient responds to the treatment. Additionally, pharmacogenomic (the effect of genotype on the pharmacokinetic, pharmacodynamic or efficacy profile of a therapeutic) information about a particular patient may affect the dosage used.

Possible pharmaceutical compositions include those suitable for oral or topical (including transdermal, buccal and sublingual) administration.

More commonly these pharmaceutical compositions are prescribed to the patient in "patient packs" containing a number of dosing units or other means for administration of metered unit doses for use during a distinct treatment period in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical composition, as herein before described, in combination with packaging material suitable for said composition. In such a patient pack the intended use of a formulation for the combination treatment can be inferred by instructions, facilities, provisions, adaptations and/or other means to help using the formulation most suitably for the treatment. Such measures make a patient pack specifically suitable for and adapted for use for treatment with the aronia extract of the present invention.

The enriched fractions may be contained in any appropriate amount in any suitable carrier substance, and it may be present in an amount of 1-99% by weight of the total weight of the composition. The composition may be provided in a dosage form that is suitable for the oral, parenteral (e.g., intravenously, intramuscularly), cutaneous, or skin (patch) administration route. Thus, the composition may be in the form of, e.g., tablets, capsules, pills, powders, granulates, suspensions, emulsions, solutions, gels including hydrogels, pastes, ointments, creams, plasters or drenches.

The pharmaceutical compositions may be formulated according to conventional pharmaceutical practice (see, e.g., Remington (2020)).

## Examples

**Example 1: Preparation of an aronia extract and the enriched fractions thereof according to the invention**

### Aronia extract

A concentrated chokeberry (*Aronia melanocarpa*) juice with an anthocyanin content of at least 0.5% at 65 Brix (cyanidin-3-O-glucoside chloride equivalent) was purchased and was then diluted in water to about 25 Brix. Diluted product was contacted with Amberlite™ XAD-7HP resin. Resin was washed using acidified water, removing most of sugars and acids. Bound phenolics were then eluted using. an acidified hydro-ethanolic solution. Elution solution was collected and concentrated by evaporation until reaching at least 45% dry matter. The concentrated elution solution was then vacuum dried at a temperature between 68°C-70°C and grinded (sieving to less than 500 $\mu$m). A red - violet to dark red powder was obtained, which is named the aronia extract or the aronia crude extract in the examples.

### Enriched fractions

To assess which polyphenol class and type, that is, anthocyanins, PACs oligomers or PACs polymers, was responsible for the effects observed in the whole aronia extract, the aronia extract was fractionated. To this aim, a gel-filtration chromatography was used, as described by Rodriguez-Daza et al. (2020) to produce three fractions:

- ACN: a fraction rich in anthocyanins and phenolic acids;
- OLI: a fraction rich in PACs oligomers, phenolic acids and flavonols, with a mean degree of polymerization DP of PACs of about 11, and
- POL: a fraction rich in PACs polymers, with a mean PACs DP of about 74.

Briefly, the aronia extract was solubilized to 75 g/L in acidified water (1% acetic acid) and was deposited on 30 g of cation exchange resin (siliaPrepX SCX, silicycle, Canada). The aronia extract was eluted with 50% ethanol (OLI fraction), and further with acidified methanol with 5% of HCl (ACN fraction). ACN fraction was then passed through XAD 7 resin and further evaporated and lyophilized. The OLI fraction was also evaporated and recovered in ethanol/water solution (50/50: v/v). The purification of the PACs-rich fraction was further performed on a Sephadex LH-20 column. Redisep column (Teledyne, Isco) containing 65 g of Sephadex LH-20 (bed volume = 100 mL) was placed on a Spot Prep system (Armen Instrument, France). The system is coupled to a UV detector set at 254 nm, and a fluorescence detector (emission wavelength: 230 nm - reception wavelength: 321 nm). The flow rate used was 5 mL/min. PACs oligomers and flavonoids were isolated by elution with methanol/ethanol 50/50 (OLI); and the PACs polymers were eluted with 70% acetone (POL).

**Example 2: Analysis of an extract according to the invention and of the enriched fractions thereof**

### Characterization of the aronia extract and fractions

*Analysis of anthocyanins by HPLC-Vis*

The method used for the quantification of anthocyanins was adapted from Dudonne *et al.* (2014) with slight modifications. The column used was a Zorbax Stablebond SB-C18 (4.6 $\times$ 250 mm, 5 $\mu$m) (Agilent Technologies, Santa

Clara, CA, USA) and the gradient was as follows : 0 - 2 min : 5% B, 2 - 10 min : 5 - 20% B, 10 - 15 min : 20% B, 15 - 30 min : 20 - 25% B, 30 - 35 min : 25% B, 35 - 50 min : 25 - 33% B, 50 - 55 min : 33% B, 55 - 65 min : 33 - 36% B, 65 - 70 min : 36 - 45% B, 70 - 75 min : 45 - 53% B, 75 - 80 min : 53 - 55% B, 80 - 84 min : 55 - 70% B, 84 - 85 min : 70 - 5% B and 85 - 90 min : 5% B. Cyanidin 3-glucoside was used for quantification. Each sample were injected in triplicate.

*Analysis of phenolic acids, flavan-3-ols, PACs and flavonols by UPLC-UV-QToF*

The method was adapted from Jakobek et al. (2013). 25 mg of sample was dissolved in 1 mL of methanol:water (80:20 v/v) acidified with 1% of formic acid. Samples were vortexed, sonicated for 15 minutes and filtered through a 0.22 $\mu$m Nylon filter (Chromatographic Specialities Inc., Brockville, ON, Canada). Samples were further diluted in methanol:water (80:20 v/v) acidified with 1% of formic acid when necessary for adequate quantification. Stock solutions of epicatechin (Sigma-Aldrich, Oakville, Canada), procyanidin B2 (Extrasynthese, Genay, France), chlorogenic acid (Sigma-Aldrich, Oakville, Canada) and quercetin (Sigma-Aldrich, Oakville, Canada) were prepared in methanol at a concentration of 10 000 ppm. Calibration curve was prepared by diluting the stock solution in methanol:water (80:20 v/v) acidified with 1% of formic acid at different concentrations (1, 2, 4, 6, 8, 10, 25, 50, 100, 150, 200, 250 and 500 ppm).

Analysis was carried out with an ACQUITY I-Class UPLC-UV coupled with a Synapt G2-Si QToF (Waters, Milford MA, USA). 1 $\mu$L were injected onto an ACQUITY Premier HSS T3 column (2.1 × 100 mm, 1.8 $\mu$m) (Waters, Milford, MA) with an ACQUITY Premier HSS T3 VanGuard FIT cartridge (2.1 × 5 mm, 1.8 $\mu$m) (Waters, Milford, MA) heated to 40 °C. Mobile phase A (water) and mobile phase B (acetonitrile) were acidified with 1% of formic acid. The gradient was as follows, with a flow rate of 0.4 mL/min: 0 - 1 min : 3% B, 1 - 15 min : 3 - 13.5% B, 19-25 min : 18.5 - 30% B, 25 - 27.9 min : 30 - 37.3% B, 27.9 - 28 min : 37.3 - 95% B, 28 - 30.9 min: 95% B, 30.9 - 31 min : 95 - 3% B and 31 - 34 min : 3% B. UV signals were acquired at 280 nm (flavan-3-ols and PAC), 320 nm (phenolic acids) and 360 nm (flavonols) with a resolution of 2.4 nm and a sampling rate of 20 Hz. Samples were kept at 4 °C in the autosampler compartment. The source parameters were as follows: capillary voltage, -2,40 kV; source temperature, 150 °C; desolvation temperature, 600 °C; cone gas flow, 50 L/h and desolvation gas, 1000 L/h. Leucine-enkephaline (200 pg/$\mu$L) was infused at a flow rate of 10 $\mu$L/min for use as internal mass standard.

First, the extract and fractions were injected in full MS in negative electrospray ionization and resolution mode (resolution ≈ 25 000) with a scan range (m/z) of 50 to 2000 and a scan time of 0.2 sec to determine the mass of each peak present in the UV chromatograms (280, 320 and 360 nm). Then, the samples were re-injected in Fast-DDA mode with an inclusion list to fragment each phenolic compound detected in the first injection. Data were acquired in negative electrospray ionization and resolution mode. The MS survey scan parameters was the same as the full MS acquisition. For each MS Survey scan, 3 MS/MS scans were performed with a scan time of 0.1 s and a collision energy ramping from 15 to 45 V. Only ions in the inclusion list within +/- 20 mDa and +/-20 s were fragmented. Compounds identification was performed using Progenesis QI v2.4 software. When authentic standard was not available, identification was done using Phenol-Explorer as database. The search was done with a precursor tolerance of 10 ppm and was filtered by an isotope similarity score higher than 90%. Theoretical fragmentation was performed with a fragment tolerance of 10 ppm. After identification, phenolic compounds were quantified using UV data with TargetLynx XS v4.2 software. Flavan-3-ols were quantified as epicatechin equivalent, phenolic acids as chlorogenic equivalent and flavanols as quercetin equivalent. Each sample were injected in triplicate.

*Determination of total PACs content and mean DP using phloroglucinolysis by UPLC-UV-QToF*

This method was also adapted from Jakobek et al. (2013). 800 $\mu$L of 0.1N HCl in methanol containing 50 g/L of phloroglucinol and 10 g/L of ascorbic acid was added to 10 mg of sample. Samples were vortexed and incubated in a water bath at 50 °C for 20 minutes. Reaction was stopped by adding 1 mL of 40 mM sodium acetate in water. For the PAC polymers fraction, the volumes of reagents were multiplied by 10. Samples were filtered using 0.22 $\mu$m PVDF filter (Chromatographic Specialities Inc., Brockville, ON, Canada).

2 $\mu$L were injected onto an ACQUITY UPLC® HSS T3 column (2.1 × 100 mm, 1.8 $\mu$m) (Waters, Milford, MA) with an ACQUITY UPLC® HSS T3 VanGuard pre-column (2.1 × 5 mm, 1.8 $\mu$m) (Waters, Milford, MA) heated to 30 °C. The mobile phases were water (A) and acetonitrile (B), both acidified with 0.1% formic acid. The gradient was as follows, with a flow rate of 0.4 mL/min: 0 - 1 min : 3% B, 1 - 2 min : 3 - 4.5% B, 2 - 8.28 min : 4.5 - 16% B, 8.28 - 14.85 min : 16 - 50% B, 14.85 - 14.9 min : 50 - 95% B, 14.9 - 16.9 min : 95% B, 16.9 - 17 min : 95 - 3% B and 17 - 20 min : 3% B. UV signals were acquired at 280 nm with a resolution of 2.4 nm and a sampling rate of 20 Hz. Samples were kept at 4 °C in the autosampler compartment and injected on the same system as the precedent section with the same source parameters. MS data were acquired by MSE in negative electrospray ionization with a scan time of 0.2 s. The scan range (m/z) was 50 to 2000 and a collision energy ramp of 20 to 60 V was applied in the high energy function. Each sample were injected in triplicate. Data was processed using TargetLynx XS v4.2 software.

Results

The compositions in polyphenols of *Aronia* extract and of polyphenolic fractions thereof are detailed in Table 1.

**Table 1: Composition in polyphenols of aronia extract and enriched fractions thereof**

| | Aronia crude extract | ACN | OLI | POL |
|---|---|---|---|---|
| | *Aronia melanocarpa* extract | anthocyanin/phenolic-rich fraction | PAC oligomers rich fraction | PAC polymers rich fraction |
| Anthocyanins | 4.97 ± 0.13 | 10.25 ± 0.63 | 0.160 ± 0.003 | 0.055 ± 0.001 |
| Flavonols | 1.82 ± 0.01 | 3.67 ± 0.02 | 0.016 ± 0.001 | ND |
| Phenolic acids | 11.46 ± 0.05 | 23.49 ± 0.10 | 0.060 ± 0.001 | 0.026 ± 0.001 |
| Catechin | 0.014 ± 0.001 | 0.033 ± 0.001 | 0.074 ± 0.001 | ND |
| Epicatechin | 0.125 ± 0.004 | 0.210 ± 0.003 | 0.431 ± 0.010 | 0.081 ± 0.002 |
| Flavan-3-ols total | 0.139 ± 0.004 | 0.242 ± 0.004 | 0.505 ± 0.010 | 0.081 ± 0.002 |
| PACs total | 21.95 ± 0.02 | 1.69 ± 0.10 | 15.15 ± 0.04 | 50.32 ± 0.05 |
| Mean DP | 29 ± 0.6 | 3 ± 0.6 | 11 ± 0.7 | 74 ± 6 |
| Values are expressed a mean value ± SD in g of polyphenols per 100 g of dry extract or fraction. Mean DP represents the mean degree of polymerization for PACs determined by phloroglucinolysis. ND: not determined, value below detection threshold. PACs: proanthocyanidins, DP: degree of polymerization. | | | | |

The detailed polyphenolic composition of the aronia extract is provided in Table 2 below.

## Example 3: antioxidant effect

The functional antioxidant effects of the aronia crude extract and the polyphenolic fractions thereof have been studied.

Direct (i.e. intracellular) antioxidant effect of each aronia extract was determined in relation to HepG2 cell line model using innovative cell-based assays for testing for intracellular radical ROS scavenging activity with AOP1 ("Anti Oxidant Power 1"), and for catalase-like activity with AOPCAT. AOP1 bioassay measures the ability of an antioxidant to neutralize oxidative stress and the effect is measured by a delay in the kinetic evolution of fluorescence emission (Gironde et al. 2020). AOP CAT is a patented technology (EP3044569). In the present analysis, it is based on intracellular presence of a nucleic acid biosensor whose fluorescence increases with $H_2O_2$. This bioassay measures the ability of an antioxidant to neutralize $H_2O_2$ (dismutation in $O_2$ and $H_2O$) and the effect is measured by a delay in the kinetic evolution of fluorescence emission. Indirect antioxidant properties (cytoprotective effects) of phytochemicals can be attributed to their ability to induce Nrf2.

*Cell culture of HepG2 cell lines*

HepG2 (passage 5 to 30) cells were cultured at 37°C in 5% $CO_2$ in Glutamax DMEM medium complemented with 10% FBS and 1× pen-strep solution. Cells were grown up to 70-80% confluence then transferred in clear bottom 96-well microplates for 24h at a density of 106 cells/mL (75 μL, 75000 cells/well).

*Determination of intracellular radical scavenging activity: AOP1 bioassay*

Aronia samples (500 mg) were solubilized at a final concentration of 50 mg/mL in the corresponding cell culture medium. Solutions were centrifuged at 8700 rpm for 10 minutes and experiments were performed using the supernatants. Cells were incubated with samples (9 different concentrations obtained by serial dilutions) for 4 hours at 37 °C in 5% CO2.

**Table 2:** Identification of phenolic compounds by UPLC-UV-QToF in crude extract and fractions

| Polyphenol class | Identification | RT (min) | [M-H]⁻ | Molecular formula | Error (ppm) | MS² fragments (Relative intensity) | ID * |
|---|---|---|---|---|---|---|---|
| Flavan-3-ols and PACs | Catechin | 7.39 | 289.0711 | C15H14O6 | -0.35 | 203.0706 (100) ; 245.0814 (57) | 1 |
| | Epicatechin | 10.81 | 289.0715 | C15H14O6 | 1.04 | 245.0814 (100) | 1 |
| | Procyanidin B2 | 9.52 | 577.1348 | C30H26O1 | 0.35 | 289.0717 (100) ; 407.0768 (87) ; 425.0872 (30) ; 245.0814 (23) | 1 |
| Phenolic acids | Neochlorogenic acid | 5.06 | 353.0873 | C16H18O9 | 0.73 | 191.0555 (100) ; 135.0447 (89) ; 179.0332 (57) | 1 |
| | Chlorogenic acid | 8.08 | 353.0871 | C16H18O9 | -0.96 | 191.0561 (100) | 1 |
| | Cryptochlorogenic acid | 8.90 | 353.0870 | C16H18O9 | -1.80 | 135.0451 (100) ; 173.0455 (67) ; 179.0350 (54) ; 191.0565 (53) | 1 |
| | Caffeoyl quinide | 12.95 | 335.0765 | C16H16O8 | -1.38 | 161.0241 (100) ; 133.0290 (50) ; 135.0444 (18) | 2 |
| | Coumaroyl quinic acid | 7.01 | 337.0918 | C16H18O8 | -1.99 | 163.0403 (100) ; 119.0502 (82) ; 191.0561 (16) | 2 |
| | Vanilloyl hexose | 7.67 | 329.0904 | C14H18O9 | -2.1 | 167.0350 (100) ; 123.0470 (34) | 2 |
| | 3,4-dihydroxybenzoic acid | 3.48 | 153.0184 | C7H6O4 | -2.61 | 109.0280 (100) | 1 |

(continued)

| Polyphenol class | Identification | RT (min) | [M-H]⁻ | Molecular formula | Error (ppm) | MS² fragments (Relative intensity) | ID * |
|---|---|---|---|---|---|---|---|
| Flavonols | Quercetin-pentoside-hexoside | 16.26 | 595.1302 | C26H2801 | -0.21 | 300.0275 (100) ; 271.0233 (14) ; 255.0286 (7) | 2 |
| | Quercetin hexoside deoxyhexoside 1 | 17.01 | 609.1432 | C27H30O1 | -1.89 | 300.0275 (100) ; 271.0233 (12) ; 255.0281 (6) | 2 |
| | Quercetin-3-galactoside | 17.27 | 463.0883 | C21H20O1 | -2.53 | 300.0275 (100) ; 271.0247 (39) ; 255.0297 (16) | 1 |
| | Quercetin-3-rutinoside | 17.51 | 609.1453 | C27H3001 | -0.98 | 300.0275 (100) ; 271.0238 (11) | 1 |
| | Quercetin-3-glucoside | 17.80 | 463.0893 | C21H20O1 | -2.24 | 300.0275 (100) ; 271.0249 (38) ; 255.0300 (16) | 1 |
| | Quercetin-dihexoside 1 | 13.88 | 625.1396 | C27H30O1 | -0.01 | 300.0275 (100) ; 271.0237 (13) | 2 |
| | Quercetin-dihexoside 2 | 14.26 | 625.1417 | C27H3001 | 0.14 | 300.0275 (100) ; 271.0243 (12) | 2 |
| | Quercetin | 23.46 | 301.0356 | C15H10O7 | 2.66 | 151,0050 (100) | 1 |
| | Quercetin-hexoside-deoxyhexoside 2 | 19.22 | 609.1449 | C27H3001 | -1.91 | 315.0477 (100) ; 314.0428 (37) ; 299.0205 (37) ; 271.0213 (24) ; | 2 |
| | Quercetin-pentoside | 18.83 | 433.0763 | C20H18O1 | -1.85 | 300.0275 (100) ; 271.0239 (55) ; 255.0292 (23) | 2 |
| | Isorhametin-hexoside-deoxyhexoside 1 | 19.83 | 623.1608 | C28H32O1 | -1.55 | 315.0511 (100) ; 314.0417 (55) | 2 |
| | Isorhametin-hexoside-deoxyhexoside 2 | 20.10 | 623.1615 | C28H32O1 | -0.30 | 315.0486 (100) ; 314.0384 (33) | 2 |
| *ID: Identification level | | | | | | | |

After the 4 hours of incubation with samples, cells were treated with thiazole orange (TO) as fluorescent biosensor for 1 hour. Fluorescence (Relative Fluorescence Unit (RFU) at 535 nm) was measured on a Varioskan equipment (Thermo Fisher Scientific, Waltham, MA USA) according to a recurrent 480 nm LED application procedure (20 iterations) of the whole 96-well plate. Raw-data kinetic profiles were recorded, and dose-response curves were modelized using Prism8 software (GraphPad, San Diego, CA, USA). The antioxidant cell index (AOP index) was calculated from normalized kinetic profiles according to the formula:

$$AOP\ index\ (\%) = 100 - 100 \left( {_0\!\int^{20} RFU_{sample}} / {_0\!\int^{20} RFU_{control}} \right)$$

Dose-response curves were obtained by compiling AOP indices according to Log of the sample concentration, and were submitted to a sigmoid fit according to the formula:

$$AOP\ index = AOP\ index_{min} + (AOP\ index_{max} - AOP\ index_{min}) / (1 + 10^{(Log(EC50-SC)*HS)})$$

where SC=sample concentration and HS=Hill slope or tangent slope at inflexion point, to calculate $EC_{50}$. Experiments were done in cell culture medium without FBS and at least two independent experiments were performed, each on triplicate wells.

*Determination of Catalase-like activity: AOP CAT bioassay*

Aronia samples (at 16 different concentrations obtained by serial dilutions) were incubated with $H_2O_2$ (0.34%) for 1 hour, and experiments were performed using the incubation compounds. The incubation compounds were added to HepG2 cells (14.7% of final volume) which were preliminarily incubated with thiazole orange (TO) as biosensor for 30 min at 37 °C in 5% $CO_2$. Fluorescence (Relative Fluorescence Unit (RFU) at 535 nm) was recorded on a kinetic mode for 75 min, with T=0 as the time of biosensor addition. Bovine liver catalase was used as positive control. Dose-response curves were modelized according to the formula:

$$CAT\text{-}like\ index = 100\ x\ [(_{35}\!\int^{75} RFU_{compound} - _{35}\!\int^{75} RFUC_{control}) / (_{35}\!\int^{75} RFU_{SAMPLE\ MAX\ control} - _{35}\!\int^{75} RFUC_{control})]$$

$EC_{50}$ values were calculated from the sigmoid fit according to the formula:

$$Fold\ Increase\ FI = FI_{min} + (FI_{max} - FI_{min})/(1 + 10^{(Log(EC50-SC)*HS)})$$

where SC=sample concentration and HS=Hill slope. Two independent experiments were performed, each on triplicate wells.

*Determination of ability to activate the Nrf2/Keap1-mediated ARE pathway: AOP2 bioassay*

ARE-Luc-HepG2 cells were incubated with samples (9 concentrations obtained by serial dilutions) for 17 h at 37 °C in 5% CO2. Luciferase assay used the BPS Bioscience (San Diego, CA, USA) ONE-Step™ Luciferase Assay System. Luminescence (RLU) was read on a Varioskan equipment (Thermo Fisher Scientific, Waltham, MA USA). Sulforaphane was used as positive control. AOP2 measures gene induction following ARE activation and results were presented as Fold Increase (FI) of gene expression according to the formula:

$$FI = (RLU_{n\ sample} / RLU_{n\ control}).$$

Dose - response curves were fitted to a sigmoid according to the formula:

$$FI = FI_{min} + (FI_{max} - FI_{min})/(1 + 10^{(Log(EC50-SC)*HS)})$$

where SC=sample concentration and HS=Hill slope, to calculate $EC_{50}$. At least two independent experiments were performed, each on duplicate wells.

*Statistical analysis*

Analysis was made using Prism8 software (GraphPad, San Diego, CA, USA). For fluorescence profiles, bars corresponded to standard error values obtained from triplicates. For dose-response figures, asymmetrical confidence intervals were reported from Prism. The two experiments that showed the best coefficient of determination ($R^2$) from the nonlinear regression model were used for EC values.

Results

*Direct antioxidant effect - AOP1 & AOPCAT*

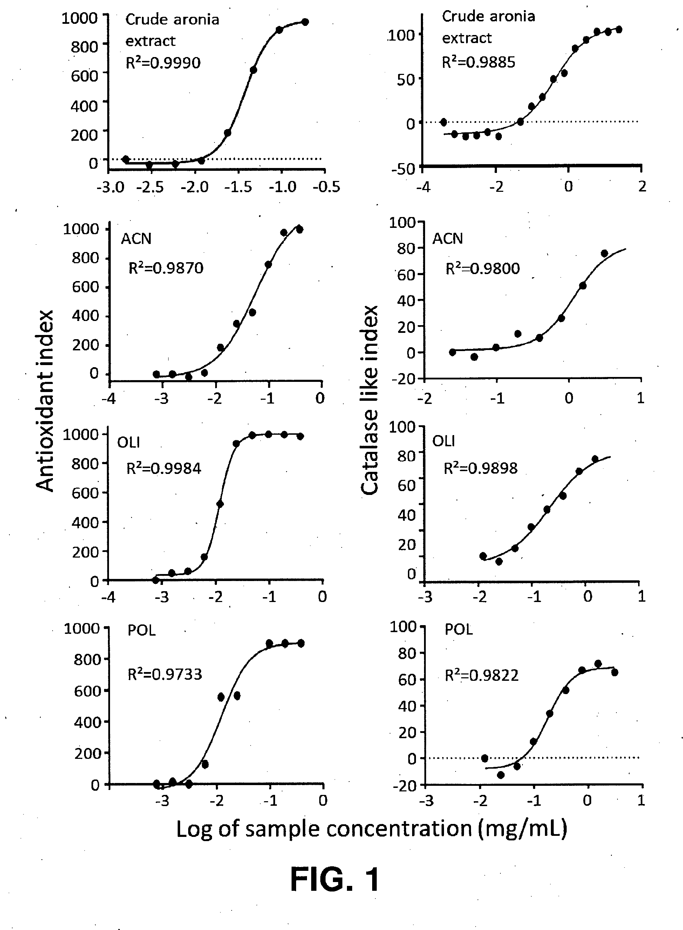

As shown in Table 3 and illustrated by Fig. 1, a direct intracellular scavenging activity of aronia crude extract was shown by the AOP1 bioassay. It was then determined in which one(s) of the purified polyphenolic fractions exert(s) this antioxidant activity. Among the aronia enriched fractions, the oligomeric OLI ($EC_{50}$ = 11.88 $\mu$g/mL) followed by the polymeric POL ($EC_{50}$= 12.36 pg/mL) PACs- rich fractions exerted the highest intracellular radical scavenging activities, conferring to the aronia crude extract its antioxidant activity ($EC_{50}$= 37.9 $\mu$g/mL). In contrast, the intracellular antioxidant activity of the anthocyanin/phenolic-rich fraction. ACN (containing phenolic acids, flavonols and anthocyanins) was weaker ($EC_{50}$= 56.46 $\mu$g/mL) and less efficient than the crude aronia extract.

A catalase-like effect was exerted by all the enriched fractions. Furthermore, the efficacy of this catalase-like activity was inversely correlated to the degree of polymerization of the PACs, as the POL fraction showed the lowest $EC_{50}$ value, followed by the OLI fraction (180.7 $\mu$g/mL. and 215.8 $\mu$g/mL, respectively). Hence, the catalase-like activity was exerted by the PACs rich fractions, more efficiently than the aronia crude extract (425.5 $\mu$g/mL) (see Fig 1, right panels), with the ACN fraction showing the lowest catalase-like activity ($EC_{50}$ =1186 $\mu$g/mL).

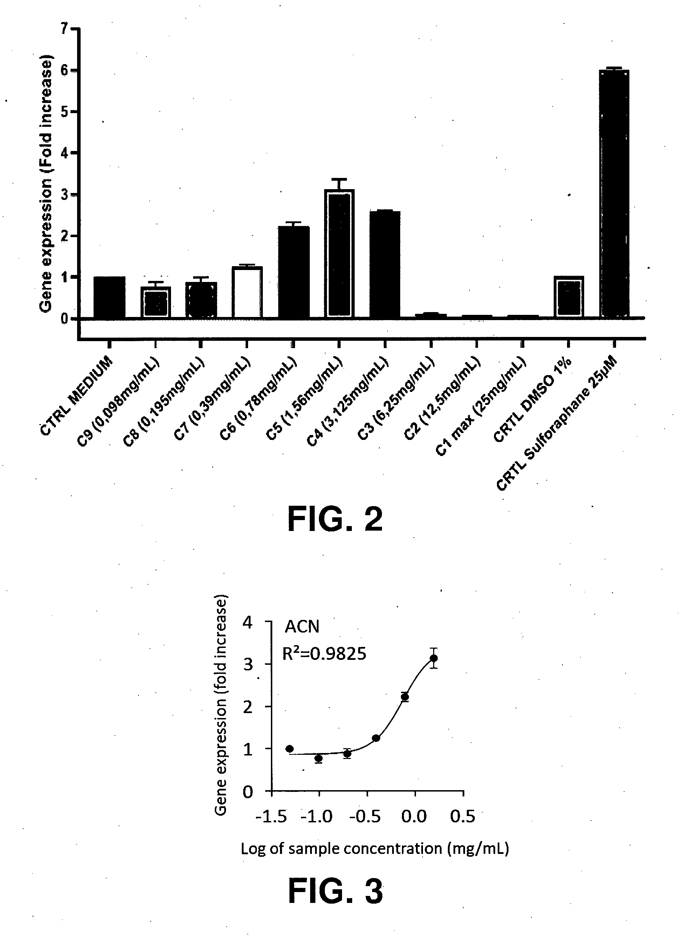

The bioassay was performed on HepG2 cells with the aronia anthocyanin fraction. Luminescence values or luciferase gene expression following ARE activation are presented as gene expression fold increase following a concentration range of the anthocyanin fraction. Results are shown in Figures. 2 and 3. The anthocyanin fraction showed a dose-dependent effect, with a fold increase (3.13 X) which was maximal at concentration of 1.56 mg/mL (C5) and with AOP2 $EC_{50}$ = 742 $\mu$g/mL. The drop of gene expression at 6.25 mg/mL (C3) can be interpreted as a toxic effect. The OLI and POL fractions did not show any indirect activity via Nrf2/Keap1 - mediated ARE induction pathway (data not shown).

**Table 3: $EC_{50}$ in HepG2 cells of the aronia crude extract and the enriched fractions ACN, OLI and POL**

| Compound | AOP1 | | | AOPCAT | | |
|---|---|---|---|---|---|---|
| | $EC_{50}$ ($\mu$g/mL) | 95% CI | Relative antioxidant effect* (%) | $EC_{50}$ ($\mu$g/mL) | 95% CI | Relative antioxidant effect* (%) |
| aronia crude extract | 37.9 | [34.9, 41.15] | 100 | 425.5 | [297.4, 640.2] | 100 |
| ACN | 55.46 | [34.8 , 166.1] | 68.34 | 1186 | [745.5, 12660] | 35.88 |
| OLI | 11.88 | [10.98, 12.81] | 319.02 | 215.8 | [124.6, 425.9] | 197.17 |
| POL | 12.36 | [6.24, 25.52] | 306.63 | 180.7 | [121.3, 273.6] | 235.47 |
| *Induction of the Nrf2/Keap1-mediated ARE pathway by the anthocyanin fraction. AOP2* | | | | | | |

*Comparison of intracellular antioxidant activity of aronia crude extract and fractions with other fruit powders and pure compounds:*

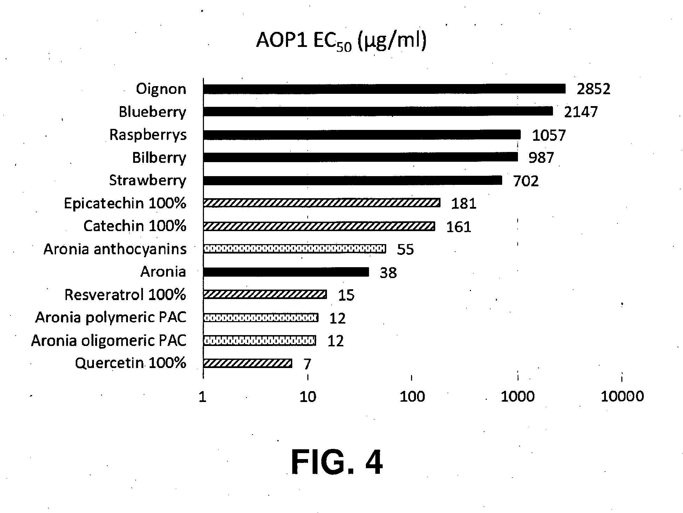

The HepG2 $EC_{50}$ for aronia crude extract was compared with data obtained for pure compounds (quercetin, resveratrol, epicatechin) and other fruit powders. The results are presented in Figure 4. aronia crude extract had a stronger intracellular antioxidant activity than epicatechin and all the tested fruit powders with various flavonoid contents: Aronia ($EC_{50}$=37.9 $\mu$g/mL) was a stronger antioxidant than strawberry ($EC_{50}$=702 $\mu$g/ml) (source of PACs), bilberry (987 $\mu$g/mL), raspberry (1057 $\mu$g/mL) and blueberry (2147 $\mu$g/mL) (sources of anthocyanins) or vegetables (onion (2852 $\mu$g/mL), source of flavonols). We then compared the $EC_{50}$ obtained for the more purified fractions of aronia with the $EC_{50}$ of pure compounds (100% quercetin, resveratrol, epicatechin) [31]. The $EC_{50}$ values of the oligomeric and polymeric PACs fractions (11.9 and 12.4 $\mu$g/mL, respectively) demonstrated that the PAC fractions exerted an intracellular antioxidant activity as strong as pure resveratrol ($EC_{50}$ resveratrol= 14.8 $\mu$g/mL). Aronia oligomeric and polymeric PACs, which are composed of epicatechin units, are more antioxidant than pure epicatechin ($EC_{50}$ epicatechin=181 $\mu$g/mL). However, pure quercetin ($EC_{50}$=7 $\mu$g/mL) was more antioxidant than the aronia PACs rich fractions.

**Conclusion**

The polyphenolic fractions exerted distinct antioxidant properties from the parent extract. Hence, the oligomeric and polymeric PACs-rich fractions (OLI and POL) showed strong intracellular radical scavenging (lowest $EC_{50}$ values) and catalase-like activities, whereas the anthocyanin/phenolic-rich fraction (ACN) showed the lowest direct antioxidant activities (highest $EC_{50}$ values). Inversely, the anthocyanin fraction (ACN) was the only *Aronia* preparation capable of activation of the Nrf2/Keap1 ARE-mediated pathway.

These examples have shown that PACs enriched fractions of aronia extract according to the invention have specific antioxidant effect directly into the cell, which is of particular interest for reducing oxidative stress, and therefore can be used for preventing, limiting, or reducing the oxidative stress due to the exposure to pollutants, aging and/or exercise, and also in the treatment of a disorder related to cellular oxidative stress, such as gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders. The anthocyanin/phenolic-rich fraction of aronia extract has no direct antioxidant effect, contrary to what was stated in some previous studies. This anthocyanin enriched fraction however has shown a great cytoprotective effect that makes it interesting for use as a cytoprotective agent, or for its a complementary action in association with the PACs enriched fractions.

**References**

Ahles S, Stevens YR, Joris PJ, Vauzour D, Adam J, de Groot E, Plat J. 2020. The Effect of Long-Term Aronia melanocarpa Extract Supplementation on Cognitive Performance, Mood, and Vascular Function: A Randomized Controlled Trial in Healthy, Middle-Aged Individuals. Nutrients 12.

Bakuradze T, Becker D, Reischmann J, Meiser P, Galan J, Richling E. 2019. Protection from DNA Damage by Use of an Aronia Food Supplement-Results from a Pilot Human Intervention Study. Current Pharmacology Reports 5:188-195.

Balentine DA, Dwyer JT, Erdman JW, Jr., Ferruzzi MG, Gaine PC, Harnly JM, Kwik-Uribe CL. 2015. Recommendations on reporting requirements for flavonoids in research. Am J Clin Nutr 101:1113-1125.

Bianco A, Dvorák A, Capková N, Gironde C, Tiribelli C, Furger C, Vitek L, Bellarosa C. 2020. The Extent of Intracellular Accumulation of Bilirubin Determines Its Anti- or Pro-Oxidant Effect. Int J Mol Sci 21.

Borowska S, Brzóska MM. 2016. Chokeberries (Aronia melanocarpa) and Their Products as a Possible Means for the Prevention and Treatment of Noncommunicable Diseases and Unfavorable Health Effects Due to Exposure to Xenobiotics. Compr Rev Food Sci Food Saf 15:982-1017.

Cassidy A, Mukamal KJ, Liu L, Franz M, Eliassen AH, Rimm EB. 2013. High anthocyanin intake is associated with a reduced risk of myocardial infarction in young and middle-aged women. Circulation 127:188-196.

Cuadrado A, et al. 2019. Therapeutic targeting of the NRF2 and KEAP1 partnership in chronic diseases. Nature Reviews Drug Discovery 18:295-317.

Denev PN, Kratchanov CG, Ciz M, Lojek A, Kratchanova MG. 2012. Bioavailability and Antioxidant Activity of Black Chokeberry (Aronia melanocarpa) Polyphenols: in vitro and in vivo Evidences and Possible Mechanisms of Action: A Review. Comprehensive Reviews in Food Science and Food Safety 11:471-489.

Derick S, Gironde C, Perio P, Reybier K, Nepveu F, Jauneau A, Furger C. 2017. LUCS (Light-Up Cell System), a universal high throughput assay for homeostasis evaluation in live cells. Scientific Reports 7:18069.

Dudonné S, Dubé P, Pilon G, Marette A, Jacques H, Weisnagel J, Desjardins Y. 2014. Modulation of Strawberry/Cranberry Phenolic Compounds Glucuronidation by Co-Supplementation with Onion: Characterization of Phenolic Metabolites in Rat Plasma Using an Optimized μSPE-UHPLC-MS/MS Method. Journal of Agricultural and Food Chemistry 62:3244-3256.

Fereidoon S, Naczk M. 1995. Food phenolics : sources, chemistry, effects, applications. Basel: Lancaster.

Forman HJ, Davies KJ, Ursini F. 2014. How do nutritional antioxidants really work: nucleophilic tone and parahormesis versus free radical scavenging in vivo. Free Radic Biol Med 66:24-35.

Fraga CG, Croft KD, Kennedy DO, Tomás-Barberán FA. 2019. The effects of polyphenols and other bioactives on human health. Food Funct 10:514-528.

Ghosh M, Kim IS, Lee YM, Hong SM, Lee TH, Lim JH, Debnath T, Lim BO. 2018. The Effects of Aronia melanocarpa 'Viking' Extracts in Attenuating RANKL-Induced Osteoclastic Differentiation by Inhibiting ROS Generation and c-FOS/NFATc1 Signaling. Molecules 23.

Gironde C, Rigal M, Dufour C, Furger C. 2020. AOP1, a New Live Cell Assay for the Direct and Quantitative Measure of Intracellular Antioxidant Effects. Antioxidants (Basel) 9.

Holmstrom KM, Finkel T. 2014. Cellular mechanisms and physiological consequences of redox-dependent signalling.

Nat Rev Mol Cell Biol 15:411-421.

Istas G, et al. 2019. Effects of aronia berry (poly)phenols on vascular function and gut microbiota: a double-blind randomized controlled trial in adult men. Am J Clin Nutr 110:316-329.

Jakobek L, Garcia-Villalba R, Tomás-Barberán FA. 2013. Polyphenolic characterisation of old local apple varieties from Southeastern European region. Journal of Food Composition and Analysis 31:199-211.

Jang BK, Lee JW, Choi H, Yim SV. 2020. Aronia melanocarpa Fruit Bioactive Fraction Attenuates LPS-Induced Inflammatory Response in Human Bronchial Epithelial Cells. Antioxidants (Basel) 9. Jurikova T, Mlcek J, Skrovankova S, Sumczynski D, Sochor J, Hlavacova I, Snopek L, Orsavova J. 2017. Fruits of Black Chokeberry Aronia melanocarpa in the Prevention of Chronic Diseases. Molecules 22.

Kardum N, Konic-Ristic A, Savikin K, Spasic S, Stefanovic A, Ivanisevic J, Miljkovic M. 2014. Effects of polyphenol-rich chokeberry juice on antioxidant/pro-oxidant status in healthy subjects. J Med Food 17:869-874.

Loo BM, Erlund I, Koli R, Puukka P, Hellström J, Wähälä K, Mattila P, Jula A. 2016. Consumption of chokeberry (Aronia mitschurinii) products modestly lowered blood pressure and reduced low-grade inflammation in patients with mildly elevated blood pressure. Nutr Res 36:1222-1230.

Ma Q. 2013. Role of nrf2 in oxidative stress and toxicity. Annu Rev Pharmacol Toxicol 53:401-426. Manach C, Williamson G, Morand C, Scalbert A, Rémésy C. 2005. Bioavailability and bioefficacy of polyphenols in humans. I. Review of 97 bioavailability studies. Am J Clin Nutr 81:230s-242s.

Meng L, Xin G, Li B, Li D, Sun X, Yan T, Li L, Shi L, Cao S, Meng X. 2018. Anthocyanins Extracted from Aronia melanocarpa Protect SH-SY5Y Cells against Amyloid-beta (1-42)-Induced Apoptosis by Regulating Ca(2+) Homeostasis and Inhibiting Mitochondrial Dysfunction. J Agric Food Chem 66:12967-12977.

Miller V, et al. 2017. Fruit, vegetable, and legume intake, and cardiovascular disease and deaths in 18 countries (PURE): a prospective cohort study. The Lancet 390:2037-2049.

Pandey KB, Rizvi SI. 2009. Plant polyphenols as dietary antioxidants in human health and disease. Oxid Med Cell Longev 2:270-278.

Paulrayer A, Adithan A, Lee JH, Moon KH, Kim DG, Im SY, Kang CW, Kim NS, Kim JH. 2017. Aronia melanocarpa (Black Chokeberry) Reduces Ethanol-Induced Gastric Damage via Regulation of HSP-70, NF-KB, and MCP-1 Signaling. Int J Mol Sci 18.

Pokimica B, García-Conesa MT, Zec M, Debeljak-Martacic J, Rankovic S, Vidovic N, Petrovic-Oggiano G, Konic-Ristic A, Glibetic M. 2019. Chokeberry Juice Containing Polyphenols Does Not Affect Cholesterol or Blood Pressure but Modifies the Composition of Plasma Phospholipids Fatty Acids in Individuals at Cardiovascular Risk. Nutrients 11.

Prior RL, Fan E, Ji H, Howell A, Nio C, Payne MJ, Reed J. 2010. Multi-laboratory validation of a standard method for quantifying proanthocyanidins in cranberry powders. J Sci Food Agric 90:1473-1478.

Rampon C, Volovitch M, Joliot A, Vriz S. 2018. Hydrogen Peroxide and Redox Regulation of Developments. Antioxidants 7:159.

Remington. 2020. Remington: The Science and Practice of Pharmacy

Rodríguez-Daza M-C, Daoust L, Boutkrabt L, Pilon G, Varin T, Dudonné S, Levy É, Marette A, Roy D, Desjardins Y. 2020. Wild blueberry proanthocyanidins shape distinct gut microbiota profile and influence glucose homeostasis and intestinal phenotypes in high-fat high-sucrose fed mice. Scientific Reports 10:2217.

Serino A, Salazar G. 2018. Protective Role of Polyphenols against Vascular Inflammation, Aging and Cardiovascular Disease. Nutrients 11.

Sidor A, Gramza-Michalowska A. 2019. Black Chokeberry Aronia Melanocarpa L.-A Qualitative Composition, Phenolic Profile and Antioxidant Potential. Molecules 24:3710.

Sies H, Berndt C, Jones D. 2017. Oxidative Stress. Annu. Rev. Biochem. 86:715-748.

Stankiewicz B, Cieslicka M, Kujawski S, Piskorska E, Kowalik T, Korycka J, Skarpanska-Stejnborn A. 2021. Effects of antioxidant supplementation on oxidative stress balance in young footballers- a randomized double-blind trial. J Int Soc Sports Nutr 18:44.

Staszowska-Karkut M, Materska M. 2020. Phenolic Composition, Mineral Content, and Beneficial Bioactivities of Leaf Extracts from Black Currant (Ribes nigrum L.), Raspberry (Rubus idaeus), and Aronia (Aronia melanocarpa). Nutrients 12.

Tebay LE, Robertson H, Durant ST, Vitale SR, Penning TM, Dinkova-Kostova AT, Hayes JD. 2015. Mechanisms of activation of the transcription factor Nrf2 by redox stressors, nutrient cues, and energy status and the pathways through which it attenuates degenerative disease. Free Radic Biol Med 88:108-146.

Tsao R. 2010. Chemistry and biochemistry of dietary polyphenols. Nutrients 2:1231-1246.

Vigliante I, Mannino G, Maffei ME. 2019. OxiCyan®, a phytocomplex of bilberry (Vaccinium myrtillus) and spirulina (Spirulina platensis); exerts both direct antioxidant activity and modulation of ARE/Nrf2 pathway in HepG2 cells. Journal of Functional Foods 61:103508.

Wang Z, Liu Y, Zhao X, Liu S, Liu Y, Wang D. 2020. *Aronia melanocarpa* Prevents Alcohol-Induced Chronic Liver Injury via Regulation of Nrf2Signaling in C57BL/6 Mice. Oxidative Medicine and Cellular Longevity 2020:4054520.

Xie L, Vance T, Kim B, Lee SG, Caceres C, Wang Y, Hubert PA, Lee JY, Chun OK, Bolling BW. 2017. Aronia berry polyphenol consumption reduces plasma total and low-density lipoprotein cholesterol in former smokers without lowering biomarkers of inflammation and oxidative stress: a randomized controlled trial. Nutr Res 37:67-77.

**Claims**

1. A proanthocyanidins (PACs) rich fraction of an aronia extract, wherein said fraction comprises:

   - an anthocyanin content of less than 5 dry wt.%, preferably less than 1 dry wt.%;
   - a flavonol content of less than 1 dry wt.%, preferably less than 0.1 dry wt.%;
   - a phenolic acid content of less than 0.1 dry wt.%;
   - a proanthocyanidin total content of at least 5 dry wt.%, preferably at least 10 dry wt.%;

   wherein the proanthocyanidin total content, the anthocyanin content, the flavonol content and the phenolic acid are measured by liquid chromatographic techniques.

2. The PACs rich fraction according to claim 1, wherein the mean degree of polymerization of PACs is comprised between 5 and 15, and wherein the fraction further comprises a flavan-3-ols total content of about 0.50 dry wt.%, as measured by liquid chromatographic techniques.

3. The PACs rich fraction according to claim 1, wherein the mean degree of polymerization of PACs is of at least 40, preferably at least 50, wherein the proanthocyanidin total content is at least 30 dry wt.%, preferably at least 40 dry wt.%, and wherein the fraction further comprises a flavan-3-ols total content of less than 0.1 dry wt.% as measured by liquid chromatographic techniques.

4. An anthocyanin/phenolic-rich fraction of an aronia extract, wherein said fraction comprises:

   - an anthocyanin content of at least 5 dry wt.%, preferably of at least 8 dry wt.%;
   - a flavonol content of at least 2.5 dry wt.%, preferably of at least 2.5 dry wt.%;
   - a phenolic acid content of at least 15 dry wt.%, preferably of at least 20 dry wt.%;
   - a flavan-3-ols total content of about 0.24 dry wt.%;
   - a proanthocyanidin total content of less than 5 dry wt.%, preferably of less than 2.5 dry wt.%; wherein the anthocyanin content, the flavonol content, the phenolic acid, the flavan-3-ols total content and the proanthocyanidin total content are measured by liquid chromatographic techniques.

5. The PACs- or anthocyanin/phenolic-rich fraction according to any of the previous claims, wherein said aronia extract is a polyphenolic aronia extract comprising proanthocyanidins (PACs) and anthocyanins, wherein:

   - the polyphenol content is at least 55 dry wt.%, preferably at least 60 dry wt.%,
   - the PACs content is at least 19 dry wt.%, preferably at least 20 dry wt.%,
   - the PACs:anthocyanins ratio is at least 1.5, and

   wherein the polyphenol content can be measured by the Folin-Ciocalteu (epicatechin equivalent), the proanthocyanidin content can be measured by the BL-DMAC method (procyanidin A2 equivalent), and the anthocyanin content can be measured by spectrophotometry (cyanidin-3-O-glucoside chloride equivalent).

6. The PACs- anthocyanin/phenolic-rich fraction according to any of the previous claims, wherein said aronia extract is selected from the group consisting of an *Aronia arbutifolia* extract, an *Aronia melanocarpa* extract and an *Aronia prunifolia* extract.

7. The PACs- or anthocyanin/phenolic-rich fraction according to claim 6; wherein the aronia extract is an extract obtained from the fruits of *Aronia melanocarpa.*

8. The PACs- or anthocyanin/phenolic-rich fraction according to any of the previous claims, wherein the extract is in a dry powder form.

9. A nutraceutical or cosmetic composition comprising, or consisting of, the PACs- or anthocyanin/phenolic- rich fraction according to any of the claims 1 to 8.

10. Use of a composition according to claim 9, wherein the enriched fraction is a PACs- rich fraction, for preventing, limiting, or reducing the oxidative stress due to the exposure to pollutants, aging and/or exercise.

11. A pharmaceutical composition comprising the PACs- or anthocyanin/phenolic-rich fraction according to any of the claims 1 to 8.

12. The pharmaceutical composition of claim 11, wherein the enriched fraction is a PACs- rich fraction, for use in the treatment of a disorder related to cellular oxidative stress in a subject in need thereof.

13. The pharmaceutical composition for use according to claim 12, wherein said disorder is selected from the group consisting of gut barrier dysfunction, intestinal inflammation, inflammatory bowel diseases, irritable bowel disease, insulin resistance, type 2 diabetes mellitus, metabolic syndrome, cardiovascular disorders, and neurodegenerative disorders.

14. The pharmaceutical composition of claim 11, wherein the enriched fraction is an anthocyanin/phenolic-rich fraction, for use as cytoprotective agent.

**FIG. 1**

## FIG. 2

FIG. 3

AOP1 EC$_{50}$ (μg/ml)

| | |
|---|---|
| Oignon | 2852 |
| Blueberry | 2147 |
| Raspberrys | 1057 |
| Bilberry | 987 |
| Strawberry | 702 |
| Epicatechin 100% | 181 |
| Catechin 100% | 161 |
| Aronia anthocyanins | 55 |
| Aronia | 38 |
| Resveratrol 100% | 15 |
| Aronia polymeric PAC | 12 |
| Aronia oligomeric PAC | 12 |
| Quercetin 100% | 7 |

1    10    100    1000    10000

**FIG. 4**

Europäisches
Patentamt

European
Patent Office

Office européen
des brevets

# EUROPEAN SEARCH REPORT

Application Number

EP 22 31 5053

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/118027 A1 (BIOVICO SP Z O O [PL]) 28 July 2016 (2016-07-28) * claims; examples * | 1-14 | INV. A61K36/73 A61P39/06 |
| X | Bräunlich Marie: "Bioactive constituents in aronia berries", , 1 January 2014 (2014-01-01), XP055945801, Retrieved from the Internet: URL:https://core.ac.uk/download/pdf/3083082 9.pdf [retrieved on 2022-07-25] * figures 8,9 * | 1-14 | |
| X | BRÄUNLICH MARIE ET AL: "Extracts, Anthocyanins and Procyanidins from Aronia melanocarpa as Radical Scavengers and Enzyme Inhibitors", NUTRIENTS, vol. 5, no. 3, 4 March 2013 (2013-03-04), pages 663-678, XP055945809, DOI: 10.3390/nu5030663 * the whole document * | 1-14 | |
| X | NIESEN SONJA ET AL: "Fractionation of Extracts from Black Chokeberry, Cranberry, and Pomegranate to Identify Compounds That Influence Lipid Metabolism", FOODS, vol. 11, no. 4, 16 February 2022 (2022-02-16), page 570, XP055946102, DOI: 10.3390/foods11040570 * the whole document * | 1-14 | TECHNICAL FIELDS SEARCHED (IPC) A61K A61P |

-/--

The present search report has been drawn up for all claims

| Place of search | Date of completion of the search | Examiner |
|---|---|---|
| Munich | 27 July 2022 | Friederich, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 241 780 A1

**Europäisches Patentamt**
**European Patent Office**
**Office européen des brevets**

# EUROPEAN SEARCH REPORT

**Application Number**

**EP 22 31 5053**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GALVÁN D'ALESSANDRO LEANDRO ET AL: "Integrated process extraction-adsorption for selective recovery of antioxidant phenolics fromAronia melanocarpaber", SEPARATION AND PURIFICATION TECHNOLOGY, vol. 120, 5 October 2013 (2013-10-05), pages 92-101, XP028785308, ISSN: 1383-5866, DOI: 10.1016/J.SEPPUR.2013.09.027 * the whole document * | 1-14 | |
| X | KOSTKA TINA ET AL: "Pomegranate (Punica granatum L.) Extract and Its Anthocyanin and Copigment Fractions—Free Radical Scavenging Activity and Influence on Cellular Oxidative Stress", FOODS, vol. 9, no. 11, 6 November 2020 (2020-11-06), page 1617, XP055828779, DOI: 10.3390/foods9111617 * figure 1 * | 1-14 | |
| X,D | SIDOR ET AL: "Black Chokeberry Aronia melanocarpa L.-A Qualitative Composition, Phenolic Profile and Antioxidant Potential", MOLECULES, vol. 24, no. 20, 15 October 2019 (2019-10-15), page 3710, XP055945768, DOI: 10.3390/molecules24203710 * the whole document * | 1-14 | **TECHNICAL FIELDS SEARCHED (IPC)** |

The present search report has been drawn up for all claims

| Place of search | Date of completion of the search | Examiner |
|---|---|---|
| Munich | 27 July 2022 | Friederich, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 22 31 5053

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-2022

| Patent document cited in search report | | Publication date | Patent family member(s) | | Publication date |
|---|---|---|---|---|---|
| WO 2016118027 | A1 | 28-07-2016 | PL | 237183 B1 | 22-03-2021 |
| | | | WO | 2016118027 A1 | 28-07-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3044569 A

**Non-patent literature cited in the description**

- **AHLES S ; STEVENS YR ; JORIS PJ ; VAUZOUR D ; ADAM J ; DE GROOT E ; PLAT J.** The Effect of Long-Term Aronia melanocarpa Extract Supplementation on Cognitive Performance, Mood, and Vascular Function: A Randomized Controlled Trial in Healthy, Middle-Aged Individuals. *Nutrients,* 2020, vol. 12
- **BAKURADZE T ; BECKER D ; REISCHMANN J ; MEISER P ; GALAN J ; RICHLING E.** Protection from DNA Damage by Use of an Aronia Food Supplement-Results from a Pilot Human Intervention Study. *Current Pharmacology Reports,* 2019, vol. 5, 188-195
- **BALENTINE DA ; DWYER JT ; ERDMAN JW, JR. ; FERRUZZI MG ; GAINE PC ; HARNLY JM ; KWIK-URIBE CL.** Recommendations on reporting requirements for flavonoids in research. *Am J Clin Nutr,* 2015, vol. 101, 1113-1125
- **BIANCO A ; DVOŘÁK A ; CAPKOVÁ N ; GIRONDE C ; TIRIBELLI C ; FURGER C ; VITEK L ; BELLAROSA C.** The Extent of Intracellular Accumulation of Bilirubin Determines Its Anti- or Pro-Oxidant Effect. *Int J Mol Sci,* 2020, vol. 21
- **BOROWSKA S ; BRZÓSKA MM.** Chokeberries (Aronia melanocarpa) and Their Products as a Possible Means for the Prevention and Treatment of Noncommunicable Diseases and Unfavorable Health Effects Due to Exposure to Xenobiotics. *Compr Rev Food Sci Food Saf,* 2016, vol. 15, 982-1017
- **CASSIDY A ; MUKAMAL KJ ; LIU L ; FRANZ M ; ELIASSEN AH ; RIMM EB.** High anthocyanin intake is associated with a reduced risk of myocardial infarction in young and middle-aged women. *Circulation,* 2013, vol. 127, 188-196
- **CUADRADO A et al.** Therapeutic targeting of the NRF2 and KEAP1 partnership in chronic diseases. *Nature Reviews Drug Discovery,* 2019, vol. 18, 295-317

- **DENEV PN ; KRATCHANOV CG ; CIZ M ; LOJEK A ; KRATCHANOVA MG.** Bioavailability and Antioxidant Activity of Black Chokeberry (Aronia melanocarpa) Polyphenols: in vitro and in vivo Evidences and Possible Mechanisms of Action: A Review. *Comprehensive Reviews in Food Science and Food Safety,* 2012, vol. 11, 471-489
- **DERICK S ; GIRONDE C ; PERIO P ; REYBIER K ; NEPVEU F ; JAUNEAU A ; FURGER C.** LUCS (Light-Up Cell System), a universal high throughput assay for homeostasis evaluation in live cells. *Scientific Reports,* 2017, vol. 7, 18069
- **DUDONNÉ S ; DUBÉ P ; PILON G ; MARETTE A ; JACQUES H ; WEISNAGEL J ; DESJARDINS Y.** Modulation of Strawberry/Cranberry Phenolic Compounds Glucuronidation by Co-Supplementation with Onion: Characterization of Phenolic Metabolites in Rat Plasma Using an Optimized μSPE-UHPLC-MS/MS Method. *Journal of Agricultural and Food Chemistry,* 2014, vol. 62, 3244-3256
- **FEREIDOON S ; NACZK M.** *Food phenolics : sources, chemistry, effects, applications.* Basel: Lancaster, 1995
- **FORMAN HJ ; DAVIES KJ ; URSINI F.** How do nutritional antioxidants really work: nucleophilic tone and para-hormesis versus free radical scavenging in vivo. *Free Radic Biol Med,* 2014, vol. 66, 24-35
- **FRAGA CG ; CROFT KD ; KENNEDY DO ; TOMÁS-BARBERÁN FA.** The effects of polyphenols and other bioactives on human health. *Food Funct,* 2019, vol. 10, 514-528
- **GHOSH M ; KIM IS ; LEE YM ; HONG SM ; LEE TH ; LIM JH ; DEBNATH T ; LIM BO.** The Effects of Aronia melanocarpa 'Viking' Extracts in Attenuating RANKL-Induced Osteoclastic Differentiation by Inhibiting ROS Generation and c-FOS/NFATc1 Signaling. *Molecules,* 2018, vol. 23
- **GIRONDE C ; RIGAL M ; DUFOUR C ; FURGER C.** AOP1, a New Live Cell Assay for the Direct and Quantitative Measure of Intracellular Antioxidant Effects. *Antioxidants (Basel),* 2020, vol. 9

- **HOLMSTROM KM ; FINKEL T.** Cellular mechanisms and physiological consequences of redox-dependent signalling. *Nat Rev Mol Cell Biol,* 2014, vol. 15, 411-421
- **ISTAS G et al.** Effects of aronia berry (poly)phenols on vascular function and gut microbiota: a double-blind randomized controlled trial in adult men. *Am J Clin Nutr,* 2019, vol. 110, 316-329
- **JAKOBEK L ; GARCIA-VILLALBA R ; TOMÁS-BARBERÁN FA.** Polyphenolic characterisation of old local apple varieties from Southeastern European region. *Journal of Food Composition and Analysis,* 2013, vol. 31, 199-211
- **JANG BK ; LEE JW ; CHOI H ; YIM SV.** Aronia melanocarpa Fruit Bioactive Fraction Attenuates LPS-Induced Inflammatory Response in Human Bronchial Epithelial Cells. *Antioxidants (Basel),* 2020, vol. 9
- **JURIKOVA T ; MLCEK J ; SKROVANKOVA S ; SUMCZYNSKI D ; SOCHOR J ; HLAVACOVA I ; SNOPEK L ; ORSAVOVA J.** Fruits of Black Chokeberry Aronia melanocarpa in the Prevention of Chronic Diseases. *Molecules,* 2017, vol. 22
- **KARDUM N ; KONIC-RISTIC A ; SAVIKIN K ; SPASIC S ; STEFANOVIC A ; IVANISEVIC J ; MILJKOVIC M.** Effects of polyphenol-rich chokeberry juice on antioxidant/pro-oxidant status in healthy subjects. *J Med Food,* 2014, vol. 17, 869-874
- **LOO BM ; ERLUND I ; KOLI R ; PUUKKA P ; HELLSTRÖM J ; WÄHÄLÄ K ; MATTILA P ; JULA A.** Consumption of chokeberry (Aronia mitschurinii) products modestly lowered blood pressure and reduced low-grade inflammation in patients with mildly elevated blood pressure. *Nutr Res,* 2016, vol. 36, 1222-1230
- **MA Q.** Role of nrf2 in oxidative stress and toxicity. *Annu Rev Pharmacol Toxicol,* 2013, vol. 53, 401-426
- **MANACH C ; WILLIAMSON G ; MORAND C ; SCALBERT A ; RÉMÉSY C.** Bioavailability and bio-efficacy of polyphenols in humans. I. Review of 97 bioavailability studies. *Am J Clin Nutr,* 2005, vol. 81, 230s-242s
- **MENG L ; XIN G ; LI B ; LI D ; SUN X ; YAN T ; LI L ; SHI L ; CAO S ; MENG X.** Anthocyanins Extracted from Aronia melanocarpa Protect SH-SY5Y Cells against Amyloid-beta (1-42)-Induced Apoptosis by Regulating Ca(2+) Homeostasis and Inhibiting Mitochondrial Dysfunction. *J Agric Food Chem,* 2018, vol. 66, 12967-12977
- **MILLER V et al.** Fruit, vegetable, and legume intake, and cardiovascular disease and deaths in 18 countries (PURE): a prospective cohort study. *The Lancet,* 2017, vol. 390, 2037-2049
- **PANDEY KB ; RIZVI SI.** Plant polyphenols as dietary antioxidants in human health and disease. *Oxid Med Cell Longev,* 2009, vol. 2, 270-278
- **PAULRAYER A ; ADITHAN A ; LEE JH ; MOON KH ; KIM DG ; IM SY ; KANG CW ; KIM NS ; KIM JH.** Aronia melanocarpa (Black Chokeberry) Reduces Ethanol-Induced Gastric Damage via Regulation of HSP-70, NF-KB, and MCP-1 Signaling. *Int J Mol Sci,* 2017, vol. 18
- **POKIMICA B ; GARCÍA-CONESA MT ; ZEC M ; DEBELJAK-MARTACIC J ; RANKOVIC S ; VIDOVIC N ; PETROVIC-OGGIANO G ; KONIC-RISTIC A ; GLIBETIC M.** Chokeberry Juice Containing Polyphenols Does Not Affect Cholesterol or Blood Pressure but Modifies the Composition of Plasma Phospholipids Fatty Acids in Individuals at Cardiovascular Risk. *Nutrients,* 2019, vol. 11
- **PRIOR RL ; FAN E ; JI H ; HOWELL A ; NIO C ; PAYNE MJ ; REED J.** Multi-laboratory validation of a standard method for quantifying proanthocyanidins in cranberry powders. *J Sci Food Agric,* 2010, vol. 90, 1473-1478
- **RAMPON C ; VOLOVITCH M ; JOLIOT A ; VRIZ S.** Hydrogen Peroxide and Redox Regulation of Developments. *Antioxidants,* 2018, vol. 7, 159
- **REMINGTON.** *Remington: The Science and Practice of Pharmacy,* 2020
- **RODRÍGUEZ-DAZA M-C ; DAOUST L ; BOUTKRABT L ; PILON G ; VARIN T ; DUDONNÉ S ; LEVY É ; MARETTE A ; ROY D ; DESJARDINS Y.** Wild blueberry proanthocyanidins shape distinct gut microbiota profile and influence glucose homeostasis and intestinal phenotypes in high-fat high-sucrose fed mice. *Scientific Reports,* 2020, vol. 10, 2217
- **SERINO A ; SALAZAR G.** Protective Role of Polyphenols against Vascular Inflammation, Aging and Cardiovascular Disease. *Nutrients,* 2018, vol. 11
- **SIDOR A ; GRAMZA-MICHALOWSKA A.** Black Chokeberry Aronia Melanocarpa L.-A Qualitative Composition, Phenolic Profile and Antioxidant Potential. *Molecules,* 2019, vol. 24, 3710
- **SIES H ; BERNDT C ; JONES D.** Oxidative Stress. *Annu. Rev. Biochem.,* 2017, vol. 86, 715-748
- **STANKIEWICZ B ; CIESLICKA M ; KUJAWSKI S ; PISKORSKA E ; KOWALIK T ; KORYCKA J ; SKARPANSKA-STEJNBORN A.** Effects of antioxidant supplementation on oxidative stress balance in young footballers- a randomized double-blind trial. *J Int Soc Sports Nutr,* 2021, vol. 18, 44
- **STASZOWSKA-KARKUT M ; MATERSKA M.** Phenolic Composition, Mineral Content, and Beneficial Bioactivities of Leaf Extracts from Black Currant (Ribes nigrum L.), Raspberry (Rubus idaeus), and Aronia (Aronia melanocarpa). *Nutrients,* 2020, vol. 12

- **TEBAY LE ; ROBERTSON H ; DURANT ST ; VITALE SR ; PENNING TM ; DINKOVA-KOSTOVA AT ; HAYES JD.** Mechanisms of activation of the transcription factor Nrf2 by redox stressors, nutrient cues, and energy status and the pathways through which it attenuates degenerative disease. *Free Radic Biol Med,* 2015, vol. 88, 108-146
- **TSAO R.** Chemistry and biochemistry of dietary polyphenols. *Nutrients,* 2010, vol. 2, 1231-1246
- **VIGLIANTE I ; MANNINO G ; MAFFEI ME.** OxiCyan®, a phytocomplex of bilberry (Vaccinium myrtillus) and spirulina (Spirulina platensis); exerts both direct antioxidant activity and modulation of ARE/Nrf2 pathway in HepG2 cells. *Journal of Functional Foods,* 2019, vol. 61, 103508
- **WANG Z ; LIU Y ; ZHAO X ; LIU S ; LIU Y ; WANG D.** <i>Aronia melanocarpa</i> Prevents Alcohol-Induced Chronic Liver Injury via Regulation of Nrf2Signaling in C57BL/6 Mice. *Oxidative Medicine and Cellular Longevity,* 2020, 4054520
- **XIE L ; VANCE T ; KIM B ; LEE SG ; CACERES C ; WANG Y ; HUBERT PA ; LEE JY ; CHUN OK ; BOLLING BW.** Aronia berry polyphenol consumption reduces plasma total and low-density lipoprotein cholesterol in former smokers without lowering biomarkers of inflammation and oxidative stress: a randomized controlled trial. *Nutr Res,* 2017, vol. 37, 67-77